# FASCICULE DE BREVET EUROPEEN

(11) **EP 1 043 343 B1**
(45) Date de publication et mention de la délivrance du brevet: **03.09.2003**
(21) Numéro de dépôt: 00400546.8
(22) Date de dépôt: 29.02.2000
(51) Int. Cl.: C08F 293/00, C08G 81/02, A61K 7/06, A61K 7/043

(54) **Composition cosmétique comprenant des polymères ayant une structure en étoiles et leur utilisation**
Zusammensetzungen für Kosmetik enthaltend sternförmige Polymerisate, und ihre Anwendung
Cosmetic composition containing star shaped polymers and their use

(30) Priorité: 06.04.1999 FR 9904255
(43) Date de publication de la demande: 11.10.2000
(73) Titulaire: L'OREAL, 75008 Paris (FR)
(72) Inventeur: Mougin, Nathalie, 75011 Paris (FR)
(74) Mandataire: Dodin, Catherine

(56) Documents cités:
- WO-A-86/00626
- DE-A- 4 328 004
- DE-A- 19 602 540
- US-A- 3 907 984
- US-A- 5 371 147
- US-A- 5 804 664

## Description

La présente invention a trait à une composition, notamment cosmétique ou pharmaceutique, comprenant dans un milieu adéquat au moins un polymère de structure ordonnée bien particulière. Ces compositions trouvent une application particulière dans le domaine du maquillage et sont susceptibles d'être appliquées sur les fibres kératiniques, notamment sur les ongles, les cils, les sourcils et les cheveux.

Les compositions à appliquer sur les fibres kératiniques, telles que les vernis à ongles ou les bases de soin des ongles, comprennent, de manière usuelle, un polymère filmogène en solution ou dispersion dans un solvant organique ou aqueux, des matières colorantes telles que colorants ou pigments, et généralement des plastifiants et des agents rhéologiques.

Parmi les principales caractéristiques que doivent posséder les vernis à ongles, on doit tout particulièrement citer l'absence d'irritation de la peau et des ongles, une application aisée, un temps de séchage rapide et l'obtention d'un film homogène et d'excellente brillance.
Par ailleurs, on recherche également des compositions conduisant à la formation de film présentant une certaine flexibilité, voire souplesse, d'où une bonne résistance à l'écaillage du vernis, notamment en cas de chocs, ainsi qu'à la formation de film suffisamment durs (dureté de surface) pour modérer, voire éviter, l'usure du film en cas de frottements.

Il est ainsi habituel d'employer, pour préparer des vernis à ongles, des matières filmogènes telles que la nitrocellulose, associée, si nécessaire, à un autre polymère tel qu'une résine acrylique ou une résine alkyde et à des plastifiants. Le rôle des plastifiants est de conférer de la souplesse au film de maquillage. En effet, l'emploi d'un polymère filmogène seul donne généralement un film rigide, cassant et fragile, qui s'use rapidement. Toutefois, l'adjonction d'un plastifiant peut parfois entraîner d'autres inconvénients tels que le jaunissement du film, l'instabilité de la composition à la lumière et/ou à la chaleur, le manque de brillant et le manque de fluidité.
Par ailleurs, la plupart des compositions connues jusqu'à présent présentent le défaut de s'user rapidement en surface, ce qui contraint l'utilisatrice à renouveler à des intervalles de temps très rapprochés, l'application d'une nouvelle couche de vernis après élimination de la couche abîmée. Parmi les solutions proposées pour remédier à cette usure, on peut noter l'incorporation, dans la composition, d'agents ayant des propriétés durcissantes du film, tels que la silice.

Par ailleurs, on connaît, par le document WO86/00626 des polymères étoiles de type acryliques, susceptibles d'être employés notamment comme agent renforçateur dans les domaines des matériaux composites, des matériaux plastiques, des revêtements pour automobiles ou camions.

On connaît également par le document US5804664 des polymères étoiles à branches multiples, comprenant au moins un polyisobutylène, et pouvant être employés comme agent modificateur de viscosité ou comme additif pour les huiles de moteur.

On constate donc que, jusqu'à présent, l'obtention d'un compromis entre souplesse/flexibilité du film d'un coté et dureté dudit film de l'autre coté, s'est révélée particulièrement compliquée. C'est ce problème qui est à la base de la présente invention.

En effet, la demanderesse s'est posé le problème de réussir l'obtention d'un film présentant une certaine flexibilité/souplesse, d'où une bonne résistance à l'écaillage, tout en étant suffisamment dur pour modérer son usure en cas de frottements.

La présente invention a donc pour objet une composition cosmétique de soin ou de maquillage des ongles, naturels ou artificiels, comprenant, dans un milieu cosmétiquement acceptable défini, au moins un polymère de structure "en étoiles" tel que défini ci-après.

Un autre objet de l'invention est un procédé de traitement cosmétique des ongles, caractérisé en ce qu'il consiste à appliquer sur ces derniers une composition cosmétique telle que définie ci-dessus.

Un autre objet de l'invention est l'utilisation d'au moins un polymère tel que ci-dessus, dans une composition cosmétique ou pour la préparation d'une composition pharmaceutique permettant l'obtention d'un film présentant une bonne résistance à l'écaillage, aux chocs, aux frottements, aux rayures et/ou aux pressions.

La demanderesse a constaté qu'en utilisant les polymères tels que revendiqués, on obtenait une composition d'application aisée, qui conduisait à un film homogène et d'excellente brillance.
Par ailleurs, le film obtenu présente une plus grande durée de vie, due à sa résistance améliorée aux agressions telles que les chocs, les frottements, les rayures, les pressions.

Les compositions selon l'invention sont facilement applicables et s'étalent aisément en particulier sur les ongles. Les compositions selon l'invention trouvent notamment une application particulièrement intéressante dans le domaine du maquillage ou du soin des fibres kératiniques, telles que les ongles, naturels ou artificiels, les cils, les sourcils, les poils et les cheveux.

La composition selon l'invention comprend donc un polymère dont la structure en "étoiles" peut être illustrée, de manière générale, par la formule suivante (I) :

A-[(M1)ₚ₁ - (M2)ₚ₂ .... (Mi)ₚⱼ]ₙ

dans laquelle :
- A représente un centre multifonctionnel, de fonctionnalité "n", n étant un entier supérieur à 2, de préférence compris entre 4 et 10,
- [(M1)ₚ₁ - (M2)ₚ₂ .... (Mi)ₚⱼ] représente une chaîne polymérique, aussi appelée "branche", constituée de monomères Mi polymérisés, identiques ou différents, ayant un indice de polymérisation pj, chaque branche étant identique ou différente, et étant greffée de manière covalente sur ledit centre A,
- i étant supérieur ou égal à 2, de préférence compris entre 2 et 10;
- pj étant supérieur ou égal à 2, de préférence compris entre 10 et 20 000.

De préférence, les chaînes polymériques se présentent sous forme de blocs, de masse moléculaire supérieure ou égale à 500, pouvant aller jusqu'à 2 000 000.

Dans un mode de réalisation préférée, le polymère utilisé dans le cadre de la présente invention peut être obtenu par polymérisation radicalaire contrôlée, également appelée polymérisation radicalaire "vivante". Cette technique permet notamment de surmonter les limitations inhérentes à la polymérisation radicalaire classique, c'est-à-dire qu'elle permet notamment de contrôler la longueur des chaînes du polymère formé, et de ce fait d'obtenir des structures blocs.

La polymérisation radicalaire contrôlée permet de réduire les réactions de désactivation de l'espèce radicalaire en croissance, en particulier l'étape de terminaison, réactions qui, dans la polymérisation radicalaire classique, interrompent la croissance de la chaîne polymérique, de façon irréversible et sans contrôle.
Afin de diminuer la probabilité des réactions de terminaison, il a été proposé de bloquer, de façon transitoire et réversible, l'espèce radicalaire en croissance, en formant des espèces actives dites "dormantes" à l'aide de liaison de faible énergie de dissociation.

On utilise des liaisons de type C― halogénure (en présence de complexe métal/ligand). On parle alors de polymérisation radicalaire par transfert d'atomes, aussi connue sous l'abréviation ATRP. Ce type de polymérisation se traduit par un contrôle de la masse des polymères formés et par un faible indice de polydispersité en poids des chaînes.
D'une manière générale, la polymérisation radicalaire par transfert d'atomes s'effectue par polymérisation :
- d'un ou plusieurs monomères polymérisables radicalairement, en présence
- d'un initiateur ayant au moins un atome ou un groupe radicalairement transférable,
- d'un composé comprenant un métal de transition, susceptible de participer à une étape de réduction avec l'initiateur et une chaîne polymérique "dormante", et
- d'un ligand, pouvant être choisi parmi les composés comprenant un atome d'azote (N), d'oxygène (O), de phosphore (P) ou de soufre (S), susceptibles de se coordonner par une liaison σ audit composé comprenant un métal de transition, ou parmi les composés comprenant un atome de carbone susceptibles de se coordonner par une liaison π ou σ audit composé comprenant un métal de transition, la formation de liaisons directes entre ledit composé comprenant un métal de transition et le polymère en formation étant évitées.
Ce procédé est en particulier illustré dans la demande WO97/18247, dont l'homme du métier pourra tirer enseignement pour préparer les polymères entrant dans le cadre de la présente invention.

La nature et la quantité des monomères, initiateur(s), composé(s) comprenant le métal de transition et ligand(s) seront choisies par l'homme du métier sur la base de ses connaissances générales, en fonction du résultat recherché.

En particulier, les monomères "M" peuvent être choisis, seuls ou en mélange, parmi les composés à insaturation éthylénique, radicalairement polymérisables, répondant à la formule : 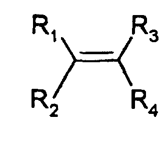 dans laquelle R₁, R₂, R₃ et R₄ sont, indépendamment les uns des autres, choisis parmi :
- un atome d'hydrogène;
- un atome d'halogène;
- un radical alkyle, linéaire ou ramifié, ayant 1 à 20, de préférence 1-6, plus préférentiellement 1-4, atomes de carbone, éventuellement substitué par un ou plusieurs halogènes et/ou un ou plusieurs radicaux -OH;
- un radical alcényle ou alkynyle, linéaire ou ramifié, ayant 2 à 10, de préférence 2-6, plus préférentiellement 2-4, atomes de carbone, éventuellement substitué par un ou plusieurs halogènes;
- un radical hydrocarboné cyclique (cycloalkyle) ayant 3 à 8 atomes de carbone, éventuellement substitué par un ou plusieurs atomes d'halogène, d'azote, de soufre, d'oxygène;
- un radical choisi parmi CN, C(=Y)R⁵, C(=Y)NR⁶R⁷, YC(=Y)R⁵, NC(=Y)R⁵ cyclique, SOR⁵, SO₂R⁵, OSO₂R⁵, NR⁸SO₂R⁵, PR⁵₂, P(=Y)R⁵₂, YPR⁵₂, YP(=Y)R⁵₂, NR⁸₂ qui peut être quatemisé avec un groupe additionnel R⁸, aryle et heterocyclyle. avec :
   - Y représente O, S ou NR⁸ (de préférence O),
   - R⁵ représente un radical alkyle, alkylthio, alcoxy, linéaire ou ramifié, ayant 1-20 atomes de carbone; un radical OH; un radical OM' avec M' = métal alcalin; un radical aryloxy ou un radical heterocyclyloxy;
   - R⁶ et R⁷ représentent, indépendamment l'un de l'autre, H ou un radical alkyle, linéaire ou ramifié, ayant 1-20 atomes de carbone; étant donné que R⁶ et R⁷ peuvent être joints pour former un groupe alkylène ayant 2-7, de préférence 2-5, atomes de carbone;
   - R⁸ représente H; un radical alkyle, linéaire ou ramifié, ayant 1-20 atomes de carbone ou un radical aryle;

- un radical -COOR dans lequel R est un radical alkyle, linéaire ou ramifié, ayant 1 à 20, de préférence 1-6, atomes de carbone, éventuellement substitué par un ou plusieurs halogènes;
- un radical -CONHR' dans lequel R' est l'hydrogène ou un radical hydrocarboné, saturé ou insaturé, linéaire ou ramifié, ayant 1 à 20, de préférence 1-6, atomes de carbone, éventuellement substitué par un ou plusieurs halogènes, azotes et/ou oxygènes;
- un radical -OCOR" dans lequel R" est l'hydrogène ou un radical hydrocarboné, saturé ou insaturé, linéaire ou ramifié, ayant 1 à 20 atomes de carbone, éventuellement substitué par un ou plusieurs halogènes, azotes et/ou oxygènes;
- un radical comprenant au moins un atome de silicium, et notamment des radicaux tels que : un radical -R-siloxane; un radical -CONHR-siloxane; un radical -COOR-siloxane ou un radical -OCO-R-siloxane, dans lesquels R est un radical alkyle, alkylthio, alcoxy, aryloxy ou hétérocycloxy, linéaire ou ramifié, ayant 1-20 atomes de carbone.

Par siloxane, on entend un composé comprenant des motifs (-SiR^{a}R^{b}O-)n, dans lesquels R^{a} et R^{b} peuvent représenter, indépendamment l'un de l'autre, un hydrogène; un halogène; un radical hydrocarboné, linéaire ou ramifié, saturé ou insaturé, ayant 1 à 36 atomes de carbone, éventuellement substitué par un ou plusieurs halogènes, azotes et/ou oxygènes; un radical hydrocarboné cyclique ayant 1 à 20 atomes de carbone; n étant supérieur ou égal à 1.
Notamment on peut citer les polydiméthylsiloxanes (PDMS) comprenant 1 à 200, de préférence moins de 100, motifs de répétition.

Par ailleurs, R¹ et R³ peuvent être reliés entre eux de manière à former un cycle de formule (CH₂)n, qui peut être substitué par un ou plusieurs halogènes et/ou oxygènes et/ou azote, et/ou par des radicaux alkyles ayant 1 à 6 atomes de carbone.

Par 'aryle' ou 'hétérocyclyle' on entend la définition communément comprise par l'homme du métier et qui peut être illustrée par l'art antérieur WO97/18247.

De préférence, on peut choisir les monomères M dans le groupe constitué par :
- les esters acryliques ou méthacryliques obtenus à partir d'alcools aliphatiques, linéaires, ramifiés, cycliques et/ou d'alcools aromatiques, de préférence en C₁-C₂₀ tel que le (méth)acrylate de méthyle, le (méth)acrylate d'éthyle, le (méth)acrylate de propyle, le (méth)acrylate de butyle, le (méth)acrylate d'isobutyle, le (méth)acrylate de tertio-butyle ;
- les (méth)acrylates d'hydroxyalkyle en C₁-C₄ tels que le (méth)acrylate de 2-hydroxyéthyle ou le (méth)acrylate de 2-hydroxypropyle;
- les (méth)acrylates d'éthylèneglycol, de diéthylèneglycol, de polyéthylèneglycol, à extrémité hydroxyle ou éther;
- les esters vinyliques, allyliques ou méthallyliques obtenus à partir d'alcools aliphatiques, linéaires ou ramifiés en C₁-C₁₀ ou cycliques en C₁-C₆ et/ou d'alcools aromatiques, de préférence en C₁-C₆, tels que l'acétate de vinyle, le propionate de vinyle, le benzoate de vinyle, le tertio-butyl benzoate de vinyle ;
- la N-vinylpyrrolidone; la vinylcaprolactame; les vinyl N-alkylpyrroles ayant 1 à 6 atomes de carbone; les vinyl-oxazoles; les vinyl-thiazoles; les vinylpyrrimidines; les vinylimidazoles; les vinyl cétones;
- les amides (méth)acryliques obtenues à partir d'amines aliphatiques, linéaires, ramifiés, cycliques et/ou d'amines aromatiques, de préférence en C₁-C₂₀ telles que le tertiobutylacrylamide; les (méth)acrylamides tels que l'acrylamide, le méthacrylamide, les dialkyl(C₁-C₄) (méth)acrylamides ;
- les oléfines tels que l'éthylène, le propylène, le styrène ou le styrène substitué;
- les monomères acryliques ou vinyliques fluorés ou perfluorés, notamment les esters (méth)acryliques à motifs perfluoroalkyle;
- les monomères comportant une fonction amine sous forme libre ou bien partiellement ou totalement neutralisée ou bien partiellement ou totalement quatemisée tels que le (méth)acrylate de diméthylaminoéthyle, le diméthylaminoéthyl méthacrylamide, la vinylamine, la vinylpyridine, le chlorure de diallyldiméthylammonium;
- les carboxybétaïnes ou les sulfobétaïnes obtenues par quatemisation partielle ou totale de monomères à insaturation éthylénique comportant une fonction amine par des sels de sodium d'acide carboxylique à halogénure mobile (chloroacétate de sodium par exemple) ou par des sulfones cycliques (propane sulfone);
- les (méth)acrylates ou (méth)acrylamides siliconés, notamment les esters(méth)acryliques à motifs siloxane;
- leurs mélanges.

Les monomères particulièrement préférés sont choisis parmi :
- les esters (méth)acryliques obtenus à partir d'alcools aliphatiques, linéaires ou ramifiés, de préférence en C₁-C₂₀;
- les esters (méth)acryliques en C₁-C₂₀ à motifs perfluoroalkyle;
- les esters(méth)acryliques en C₁-C₂₀ à motifs siloxane;
- les amides (méth)acryliques obtenues à partir d'amines aliphatiques, linéaires, ramifiés, cycliques et/ou d'amines aromatiques, de préférence en C₁-C₂₀ telles que le tertiobutylacrylamide; les (méth)acrylamides tels que l'acrylamide, le méthacrylamide, les dialkyl(C₁-C₄) (méth)acrylamides ;
- les esters vinyliques, allyliques ou méthallyliques obtenus à partir d'alcools aliphatiques, linéaires ou ramifiés en C₁-C₁₀ ou cycliques en C₁-C₆ ;
- la vinylcaprolactame ;
- le styrène éventuellement substitué;
- leurs mélanges.

Dans le cadre de la présente invention, l'initiateur peut être tout composé, notamment moléculaire ou polymérique, ayant au moins deux atomes et/ou groupes radicalairement transférables par polymérisation.
Notamment, l'initiateur peut être un oligomère ou un polymère susceptible d'être obtenu par polymérisation radicalaire, par polycondensation, par polymérisation anionique ou cationique, ou par ouverture de cycles.
Lesdits atomes et/ou groupes transférables peuvent être situés aux extrémités de la chaîne polymérique ou le long du squelette.

En particulier, on peut citer les composés correspondant à l'une des formules suivantes :
- R¹¹ₓ R¹²_{y} R¹³_{z} C-(RX)ₜ dans laquelle x, y et z représentent un entier allant de 0 à 4, t un entier allant de 1 à 4, et x+y+z= 4-t;
- R¹³ₓ C₆-(RX)_{y} (cycle à 6 carbones, saturé) dans laquelle x représente un entier allant de 7 à 11, y représente un entier allant de 1 à 5, et x+y= 12 ;
- R¹³ₓ C₆-(RX)_{y} (cycle à 6 carbones, insaturé) dans laquelle x représente un entier allant de 0 à 5, y représente un entier allant de 1 à 6, et x+y= 6;
- -[-(R¹¹)(R¹²)(R¹³) C-(RX)-]ₙ dans laquelle n est supérieur ou égal à 1; cyclique ou linéaire;
- -[-(R¹²)ₓ C₆(RX)_{y}- R¹¹-]ₙ dans laquelle x représente un entier allant de 0 à 6, y représente un entier allant de 1 à 6, et n est supérieur ou égal à 1, avec x+y=4 ou 6; cyclique ou linéaire;
- -[-(R¹²)ₓ C₆ (RX)_{y}- R¹¹-]ₙ dans laquelle x représente un entier allant de 0 à 12, y représente un entier allant de 1 à 12, et n est supérieur ou égal à 1, avec x+y=10 ou 12; cyclique ou linéaire;
- -[OSi (R¹¹)ₓ(RX)_{y}]ₙ, cyclique ou linéaire, dans laquelle x et y représentent un entier allant de 0 à 2, et n est supérieur ou égal à 1, avec x+y=2;
- R¹¹N-X₂
- (R¹¹)ₓP(O)_{y}-X₃₋ₓ dans laquelle x et y représentent des entiers allant de 0 à 2, et x+y = 5;
- (R¹¹O)ₓP(O)_{y}-X₃₋ₓ dans laquelle x et y représentent des entiers allant de 0 à 2, et x+y = 5;
- -[(R¹¹)ₜN_{z}P(O)ₓ(O-RX)_{y}-]ₙ, cyclique ou linéaire, dans laquelle x représente un entier allant de 0 à 4, y représente un entier allant de 1 à 5, z représente un entier allant de 0 à 2 et t représente un entier allant de 0 à 3, et n est supérieur ou égal à 1;
dans lesquelles :
- R, R¹¹, R¹² et R¹³ représentent, indépendamment l'un de l'autre, un atome d'hydrogène ou d'halogène; un radical alkyle, linéaire ou ramifié, ayant 1-20, de préférence 1-10 et plus préférentiellement 1-6 atomes de carbone; un radical cycloalkyle ayant 3-8 atomes de carbone; un radical -C(=Y)R⁵, -C(=Y)NR⁶R⁷ ou -R⁸₃Si (voir les définitions de R⁵ à R⁸ ci-dessus); -COCI, -OH, -CN, un radical alkényle ou alkynyle ayant 2-20, de préférence 2-6, atomes de carbone; un radical oxiranyle, glycidyle, alkylène ou alkénylène substitué avec un oxiranyle ou un glycidyle; un radical aryle, heterocyclyle, aralkyle, aralkenyle; un radical alkyle ayant 1-6 atomes de carbone dans lequel tout ou partie des atomes d'hydrogène sont substitués soit par des atomes d'halogènes tels que fluor, chlore ou brome, soit par un groupe alcoxy ayant 1-4 atomes de carbone ou par un radical aryle, heterocyclyle, -C(=Y)R⁵, -C(=Y)NR⁶R⁷, oxiranyle, glycidyle;
- X représente un atome d'halogène tel que Cl, Br, I, ou un radical -OR', -SR, -SeR, -OC(=O)R', -OP(=O)R', -OP(=O)(OR')₂, -OP(=O)OR', -O-NR'₂, -S-C(=S)NR'₂, -CN, -NC, -SCN, -CNS, -OCN, -CNO et -N₃, dans lequel R' représente un radical alkyle ayant 1-20 atomes de carbone éventuellement substitué par un ou plusieurs atomes d'halogènes notamment de fluor et/ou de chlore; et R représente un radical aryle ou alkyle, linéaire ou ramifié, ayant 1-20, de préférence 1-10, atomes de carbone; le groupement -NR'₂ pouvant en outre représenter un groupement cyclique, les deux groupes R' étant joints de manière à former un hétérocycle à 5, 6 ou 7 membres.

De préférence, X représente un atome d'halogène et notamment un atome de chlore ou de brome.

De préférence, on choisit l'initiateur parmi les composés de formule
- R¹³ₓ C₆-(RX)_{y} (cycle à 6 carbones, saturé) dans laquelle x représente un entier allant de 7 à 11, y représente un entier allant de 1 à 5, et x+y= 12 ;
- -[-(R¹²)ₓ C₆(RX)_{y}- R¹¹-]ₙ dans laquelle x représente un entier allant de 0 à 6, y représente un entier allant de 1 à 6, et n est supérieur ou égal à 1, avec x+y=4 ou 6; cyclique ou linéaire; et
- -[OSi (R¹¹)ₓ(RX)_{y}]ₙ, cyclique ou linéaire, dans laquelle x et y représentent un entier allant de 0 à 2, et n est supérieur ou égal à 1, avec x+y=2.

En particulier, on peut citer comme initiateur, les composés suivants :
- l'octa-2-isobutyrylbromide-octatertiobutyl-calix(8)arène,
- l'octa-2-propionylbromide -octatertiobutyl-calix(8)arène, et
- l'hexakis α-bromométhylbenzène.

Le composé comprenant un métal de transition, susceptible de participer à une étape de réduction avec l'initiateur et une chaîne polymérique "dormante", peut être choisi parmi ceux qui correspondent à la formule Mⁿ⁺X'ₙ, dans laquelle :
- M peut être choisi parmi Cu, Au, Ag, Hg, Ni, Pd, Pt, Rh, Co, Ir, Fe, Ru, Os, Re, Mn, Cr, Mo, W, V, Nb, Ta et Zn,
- X' peut représenter un halogène (brome ou chlore notamment), OH, (O)_{1/2}, un radical alcoxy ayant 1-6 atomes de carbone, (SO₄)_{1/2}, (PO₄)_{1/3}, (HPO₄)_{1/2}, (H₂PO₄), un radical triflate, hexafluorophosphate, methanesulfonate, arylsulfonate, SeR, CN, NC, SCN, CNS, OCN, CNO, N₃ et R'CO₂, dans lequel R représente un radical aryle ou alkyle, linéaire ou ramifié, ayant 1-20, de préférence 1-10 atomes de carbone, et R' représente H ou un radical alkyle, linéaire ou ramifié, ayant 1-6 atomes de carbone, ou un radical aryle, éventuellement substitué par un ou plusieurs atomes d'halogènes notamment de fluor et/ou de chlore;
- n est la charge du métal.

De préférence, on choisit M représentant le cuivre ou le ruthénium, et X' représentant le brome ou le chlore.
En particulier, on peut citer le bromure de cuivre.

Parmi les ligands susceptibles d'être utilisés dans le cadre de la présente invention, on peut citer les composés comprenant au moins un atome d'azote, d'oxygène, de phosphore et/ou de soufre, susceptibles de se coordonner par une liaison σ au composé comprenant un métal de transition.
On peut aussi citer les composés comprenant au moins deux atomes de carbone susceptibles de se coordonner par une liaison π audit composé comprenant un métal de transition.
On peut encore citer les composés comprenant au moins un atome de carbone susceptibles de se coordonner par une liaison σ audit composé comprenant un métal de transition, mais qui ne forment pas de liaison carbone-carbone avec le monomère lors de la polymérisation, c'est-à-dire qui ne participent pas à des réactions de β-addition avec les monomères.
On peut encore citer les composés susceptibles de se coordonner par une liaison µ ou η audit composé comprenant un métal de transition.

Notamment, on peut citer les composés de formule : R⁹-Z-(R¹⁴-Z)ₘ-R¹⁰ dans laquelle :
- R⁹ et R¹⁰ sont, indépendamment l'un de l'autre, un atome d'hydrogène; un radical alkyle, linéaire ou ramifié, ayant 1-20, de préférence 1-10 atomes de carbone; un radical aryle; un radical hétérocyclyle; un radical alkyle ayant 1-6 atomes de carbone substitué avec un radical alcoxy ayant 1-6 atomes de carbone ou un radical dialkylamino ayant 1-4 atomes de carbone ou un radical -C(=Y)R⁵ ou -C(=Y)NR⁶R⁷ et/ou YC(=Y)R⁸ (voir les définitions de R⁵ à R⁸ et Y ci-dessus); étant donné que R⁹ et R¹⁰ peuvent être joints de manière à former un cycle, saturé ou insaturé;
- R¹⁴ représente, indépendamment les uns des autres, un groupe divalent choisi parmi les alcanediyles ayant 2-4 atomes de carbone; les alkénylènes ayant 2-4 atomes de carbone; les cycloalcanediyles ayant 3-8 atomes de carbone; les cycloalkènediyles ayant 3-8 atomes de carbone; les arènediyles et les hétérocyclylènes;
- Z représente O, S, NR¹⁵ ou PR¹⁵ avec R¹⁵ représentant H; un radical alkyle, linéaire ou ramifié, ayant 1-20 atomes de carbone; un radical aryle; un radical heterocyclyle; un radical alkyle ayant 1-6 atomes de carbone substitué avec un radical alcoxy ayant 1-6 atomes de carbone ou un radical dialkylamino ayant 1-4 atomes de carbone ou un radical -C(=Y)R⁵ ou -C(=Y)NR⁶R⁷ et/ou YC(=Y)R⁸ (voir les définitions de R⁵ à R⁸ et Y ci-dessus);
- m est compris entre 0 et 6.

On peut également citer les composés de formule : R²⁰R²¹C[C(=Y)R⁵] dans laquelle :
- R²⁰ et R²¹ sont, indépendamment l'un de l'autre, un atome d'hydrogène; un atome d'halogène; un radical alkyle, linéaire ou ramifié, ayant 1-20, de préférence 1-10 atomes de carbone; un radical aryle; un radical hétérocyclyle; étant donné que R²⁰ et R²¹ peuvent être joints de manière à former un cycle, saturé ou insaturé; étant donné que chaque radical peut en outre être substitué avec un radical alkyle ayant 1-6 atomes de carbone, un radical alcoxy ayant 1-6 atomes de carbone ou un radical aryle;
- R⁵ et Y étant définis ci-dessus.

On peut encore citer comme ligands, le monoxyde de carbone; les porphyrines et les porphycènes, éventuellement substitués; l'éthylènediamine et le propylènediamine, éventuellement substitués; les multiamines avec amines tertiaires telles que le pentaméthyldiéthylènetriamine; les aminoalcools tels que l'aminoéthanol et l'aminopropanol, éventuellement substitués; les glycols tels que l'éthylèneglycol ou le propylèneglycol, éventuellement substitués; les arènes tels que le benzène, éventuellement substitués; le cyclopentadiène, éventuellement substitué; les pyridines et bipyridines, éventuellement substituées; l'acétonitrile; la 1,10-phénanthroline; les cryptands et les éthers-couronnes; la spartéine.

Les ligands préférés sont choisis notamment parmi les pyridines et bipyridines, éventuellement substituées par des radicaux alkyls en C2-C15, en particulier en C6-C12, et notamment le radical nonyle; les multiamines avec amines tertiaires telles que la pentaméthyldiéthylènetriamine.

La polymérisation des monomères, en présence de l'initiateur, du composé comprenant un métal de transition et du ligand qui joue le rôle d'activateur, conduit à l'obtention d'un polymère ayant une structure d'étoiles, qui peut être représentée par la formule (I) donnée ci-dessus, dans laquelle les monomères se sont polymérisés pour donner "n" chaînes polymériques, semblables ou différentes, toutes reliées à un centre multifonctionnel A qui dérive de l'initiateur.

On a constaté que pour atteindre le but poursuivi par la présente invention, c'est-à-dire obtenir une composition qui ne présente pas les inconvénients de l'art antérieur et qui soit en particulier souple et flexible tout en étant dure, donc qui soit à la fois résistante à l'écaillage et au frottement, il était nécessaire de choisir un polymère répondant aux critères suivants :
- il doit comprendre un ou plusieurs monomères Mi dont l'homopolymère correspondant présente une Tg supérieure ou égale à environ 0°C, de préférence supérieure ou égale à 5°C, et encore mieux supérieure ou égale à 10°C;
- ce ou ces monomères Mi étant présents, dans le polymère final, en une quantité d'environ 55-98% en poids, de préférence en une quantité de 75-95% en poids, et encore mieux en une quantité de 80-90% en poids, par rapport au poids total de monomères ;
et
- le polymère doit par ailleurs comprendre un ou plusieurs monomères Mj dont l'homopolymère correspondant présente une Tg inférieure ou égale à environ 0°C, de préférence inférieure ou égale à -5°C, et encore mieux inférieure ou égale à -10°C,
- ce ou ces monomères Mj étant présents, dans le polymère final, en une quantité d'environ 2-45% en poids, de préférence en une quantité de 5-25% en poids, et encore mieux en une quantité de 10-20% en poids, par rapport au poids total de monomères.

La mesure de Tg (température de transition vitreuse) est effectuée par DSC (Differential Scanning Calorimetry) selon la norme ASTM D3418-97.

On peut ainsi obtenir de petits nodules souples, à partir des monomère Mj, qui seront présents à l'intérieur d'une matrice polymérique filmogène, obtenue à partir des monomères Mi.

Par ailleurs, dans un mode préféré de réalisation de l'invention, au moins un des monomères Mi ou Mj comporte des motifs hydrophiles, tels que des motifs acide, alcool, amine et/ou amide, ce qui permet d'améliorer encore l'adhésion du polymère sur le support.

Les polymères tels que définis dans la présente invention doivent être filmogènes ou peuvent être rendus filmogènes par addition d'un agent auxiliaire de filmification. Par filmogène, on entend que le polymère, après application sur un support et évaporation du solvant (aqueux ou organique) conduit à un film transparent et non craquelé.
Un tel agent auxiliaire de filmification peut être choisi parmi tous les composés connus de l'homme du métier comme étant susceptibles de remplir la fonction recherchée, et peut être notamment choisi parmi les agents plastifiants et/ou parmi les agents de coalescence. En particulier, on peut citer, seuls ou en mélange :
- les glycols et leurs dérivés tels que le diéthylène glycol éthyléther, le diéthylène glycol méthyléther, le diéthylène glycol butyléther, le diéthylène glycol hexyléther, l'éthylène glycol éthyléther, l'éthylène glycol butyléther, l'éthylène glycol hexyléther;
- les esters de glycérol, tels que le diacétate de glycérol (diacétine) et le triacétate de glycérol (triacétine);
- les dérivés de propylène glycol et en particulier le propylène glycol phényléther, le propylène glycol diacétate, le propylène glycol méthyléther, le propylène glycol éthyléther, le propylène glycol butyléther, le dipropylène glycol méthyléther, le dipropylène glycol butyléther, le dipropylène glycol éthyléther, le tripropylène glycol butyléther, le tripropylène glycol méthyléther;
- des esters d'acides notamment carboxyliques, tels que des citrates, des phtalates, des adipates, des carbonates, des tartrates, des phosphates, des sébaçates,
- des dérivés oxyéthylénés tels que les huiles oxyéthylénées, notamment les huiles végétales telles que l'huile de ricin; les huiles de silicone oxyéthylénées.
La quantité d'agent auxiliaire de filmification peut être choisie par l'homme du métier sur base de ses connaissances générales, de manière à un film ayant les propriétés mécaniques souhaitées, tout en conservant à la composition des propriétés cosmétiquement acceptables.

Dans un mode de réalisation préféré de l'invention, on choisit un polymère, éventuellement en association avec des agents auxiliaires de filmification, permettant l'obtention d'un film ayant une dureté de surface inférieure à 110, de préférence comprise entre 1 et 70 et encore mieux comprise entre 5 et 55. La méthode de mesure de la dureté est décrite avant les exemples.

Les polymères tels que ci-dessus définis peuvent être présents dans le milieu sous forme dissoute ou en dispersion, dans une phase aqueuse, organique ou hydroorganique notamment alcoolique ou hydroalcoolique, et/ou une phase grasse, selon l'application envisagée.
Lesdits polymères peuvent être présents dans les compositions selon l'invention en une quantité aisément déterminable par l'homme du métier selon l'application envisagée, et qui peut être comprise entre 1 à 60% en poids de matière sèche, par rapport au poids total de la composition, de préférence entre 1 et 50% en poids et préférentiellement entre 5 et 40% en poids.

Les compositions cosmétiques ou pharmaceutiques selon l'invention comprennent donc en outre un milieu cosmétiquement ou pharmaceutiquement acceptable, qui peut être choisi par l'homme du métier selon l'application envisagée.

Ce milieu peut comprendre une phase aqueuse, une phase grasse et/ou une phase organique.
La phase aqueuse peut comprendre de l'eau et/ou une eau thermale et/ou une eau de source et/ou une eau minérale et/ou une eau florale.
Elle peut également comprendre un ou plusieurs solvants organiques cosmétiquement acceptables ou bien un mélange d'eau et d'un ou plusieurs solvants organiques cosmétiquement acceptables. Parmi ces solvants organiques, on peut citer :
- les alcools liquides à 25°C, notamment les alcools en C₁-C₄ tels que l'éthanol, l'isopropanol, le n-propanol, le diacétone alcool, le 2-butoxyéthanol, le cyclohexanol ;
- les cétones liquides à 25°C, telles que la méthyléthylcétone, la méthylisobutyl-cétone, la diisobutylcétone, l'isophorone, la cyclohexanone, l'acétone ;
- les esters d'acide carboxylique inférieurs en C₁-C₈ tels que l'acétate de méthyle, l'acétate d'éthyle, l'acétate de propyle, l'acétate de n-butyle, l'acétate d'isopentyle.
- les éthers liquides à 25°C tels que le diéthyléther, le diméthyléther ou le dichlorodiéthyléther ;
- les alcanes liquides à 25°C que le décane, l'heptane, le dodécane, le cyclohexane;
- les composés cycliques aromatiques liquides à 25°C tels que le toluène et le xylène ;
- les aldéhydes liquides à 25°C tels que le benzaldéhyde, l'acétaldéhyde;
- leurs mélanges.

La composition peut en outre comprendre une phase grasse qui peut comprendre des huiles, volatiles ou non, des gommes et/ou des cires usuelles, d'origine animale, végétale, minérale ou synthétique, seules ou en mélanges, et notamment :
- des huiles de silicone, volatiles ou non, linéaires, ramifiées ou cycliques, éventuellement organomodifiées; des silicones phénylées; des résines et des gommes de silicone liquides à température ambiante;
- des huiles minérales telles que l'huile de paraffine et de vaseline,
- des huiles d'origine animale telles que le perhydrosqualène, la lanoline;
- des huiles d'origine végétale telles que les triglycérides liquides, par exemple les huiles de tournesol, de maïs, de soja, de jojoba, de courge, de pépins de raisin, de sésame, de noisette, d'abricot, de macadamia, d'avocat, d'amande douce, de ricin, les triglycérides des acides caprylique/caprique; l'huile d'olive, l'huile d'arachide, l'huile de colza, l'huile de coprah;
- des huiles de synthèse telles que l'huile de purcellin, les isoparaffines; les alcools gras; les esters d'acides gras;
- des huiles fluorées et perfluorées; des huiles de silicones fluorées;
- des cires choisies parmi les cires animales, fossiles, végétales, minérales ou de synthèse connues, telles que les cires de paraffine, les cires de polyéthylène, les cires de Camauba, de Candellila; les cires d'abeilles; la cire de lanoline, les cires d'insectes de Chine, la cire de riz, la cire d'Ouricurry, la cire d'Alfa, la cire de fibres de liège, la cire de canne à sucre, la cire du Japon, la cire de sumac, la cire de montan, les cires microcristallines, l'ozokérite, les cires obtenues par la synthèse de Fisher-Tropsch; les cires de silicone; leurs mélanges.

Selon l'application envisagée, l'homme du métier saura déterminer la nature et al quantité de chacun des constituants. A titre indicatif, lorsque la composition comprend un milieu solvant organique, ledit solvant organique peut être présent en une teneur de 25 à 95% en poids, par rapport au poids total de la composition, de préférence de 60-90% en poids.

La composition peut en outre comprendre au moins un colorant hydrosoluble et/ou au moins un pigment, utilisés de manière usuelle dans le domaine de la cosmétique et du maquillage. Par pigments, il faut comprendre des particules blanches ou colorées, minérales ou organiques, insolubles dans le milieu, destinées à colorer et/ou opacifier la composition. Les pigments peuvent être présents dans la composition à raison de 0-20% en poids de la composition finale, et de préférence à raison de 1-5%. Ils peuvent être blancs ou colorés, minéraux et/ou organiques, de taille usuelle ou nanométrique. On peut citer, parmi les pigments et nanopigments minéraux, les oxydes de titane, de zirconium ou de cérium, ainsi que les oxydes de zinc, de fer ou de chrome, le bleu ferrique. Parmi les pigments organiques, on peut citer le noir de carbone, et les laques de baryum, strontium, calcium, aluminium. Parmi les colorants hydrosolubles, on peut citer les colorants usuels du domaine considéré tels que le sel disodique de ponceau, le sel disodique du vert d'alizarine, le jaune de quinoléine, le sel trisodique d'amarante, le sel disodique de tartrazine, le sel monosodique de rhodamine, le sel disodique de fuchsine, la xanthophylle.

Par ailleurs, la composition selon l'invention peut contenir des adjuvants couramment utilisés dans les compositions cosmétiques ou pharmaceutiques, tels que :
- des actifs cosmétiques et/ou pharmaceutiques tels que les adoucissants, les antioxydants, les opacifiants, les émollients, les hydroxyacides, les agents anti-mousse, les hydratants, les vitamines, les parfums, les conservateurs, les séquestrants, des filtres UV, des céramides; des agents anti-radicaux libres; des complexants; des absorbeurs d'odeur; des actifs de soin; des agents anti-chute des cheveux; des agents antifongiques ou antiseptiques; des anti-bactériens;
- des charges, des nacres, des laques; des épaississants, des gélifiants; des polymères notamment fixants ou conditionneurs; des agents propulseurs, des agents alcalinisants ou acidifiants; des plastifiants; des tensioactifs; des agents d'étalement; des agents mouillants; des agents dispersants;
- des polymères hydrophiles additionnels, tels que les alcools polyvinyliques et leurs copolymères; les polysaccharides ou les polymères cellulosiques; les protéines naturelles ou les polypeptides synthétiques;
- des polymères hydrosolubles;
- d'autres polymères filmogènes tels que les résines alkydes, acryliques et/ou vinyliques, les polyuréthannes et les polyesters, les celluloses et dérivés cellulosiques telles que la nitrocellulose, et les résines résultant de la condensation de formaldéhyde avec une arylsulfonamide; des polymères filmogènes en dispersion aqueuse tels que les polyuréthannes, les polyesters-polyuréthannes, les polyéther-polyuréthannes, les polymères radicalaires notamment de type acrylique, acrylique styrène et/ou vinylique, les polyesters, les résines alkydes; leurs mélanges.

Bien entendu, l'homme du métier veillera à choisir ce ou ces éventuels adjuvants, et/ou leur quantité, de manière telle que les propriétés avantageuses de la composition selon l'invention ne soient pas, ou substantiellement pas, altérées par l'adjonction envisagée.

Les compositions selon l'invention peuvent se présenter sous différentes formes et en particulier sous forme d'émulsions huile-dans-eau ou eau-dans-huile; de dispersions aqueuses, huileuses ou en milieu solvant; de solutions aqueuses, huileuses ou en milieu solvant; sous forme fluide, épaissie ou gélifiée, semi-solide, pâte souple; sous forme solide telle que de stick ou bâton.

De préférence, elles se présentent sous forme de solutions ou de dispersions aqueuses ou en milieu solvant organique, éventuellement épaissies.

Les compositions selon l'invention trouvent une application dans un grand nombre de traitements cosmétique ou pharmaceutique de la peau, des cheveux, des cils, des sourcils, des ongles, des muqueuses, du cuir chevelu.

Elles trouvent une application toute particulière en tant que produit de maquillage ou de soin des fibres kératiniques, et notamment des ongles, naturels ou artificiels, des cils, des sourcils, des poils et des cheveux.
En particulier, elles peuvent se présenter sous la forme d'une base de soin pour les ongles, d'un vernis à ongles, d'un mascara pour les cils et/ou les sourcils, d'un mascara pour les cheveux, d'une composition capillaire de soin ou de coiffage.

L'invention est illustrée plus en détail dans les exemples suivants.

### A/ Mesure de la dureté

La dureté du film est mesurée selon la norme ASTM D-43-66, ou la norme NF-T 30-016 (octobre 1981), à l'aide d'un pendule de Persoz.
Le film déposé sur le support doit avoir une épaisseur d'environ 300 microns avant séchage. Après séchage pendant 24 heures, à 30°C et sous une humidité relative de 50%, on obtient un film ayant une épaisseur d'environ 100 microns; on mesure alors sa dureté à 30°C et 50% d'humidité relative.

### Exemple 1 : Préparation de l'initiateur

L'initiateur préparé est le 5,11,17,23,29,35,41,47-octa-2-propionylbromide-49,50,51,52,53,54,55,56-octatertiobutyl-calix(8)arène (M = 2378 g).

Les réactifs utilisés sont les suivants :
- 4-tertiobutyl-calix(8)arène (M = 1298g) comportant 8 motifs phénols (Aldrich) 15 g
- 2-bromopropionylbromide de formule CH₃-CHBr-COBr 59,9 g
- triéthylamine 28 g
- tétrahydrofurane (THF) 120 g

Dans un ballon muni d'agitation et d'un thermomètre, on ajoute le 4-tbutyl-calix(8)arène et le solvant THF; on laisse sous agitation pendant 10 minutes à température ambiante.
On ajoute ensuite la triéthylamine, ce qui prend environ 15 minutes.
On ajoute alors le 2-bromopropionylbromide préalablement dissous dans le THF, à une température de 5°C environ, ce qui prend 1h30 environ.
On laisse sous agitation durant 12 heures au moins, à 5°C, puis on laisse progressivement remonter la température jusqu'à température ambiante.
On concentre la solution obtenue par évaporation du THF. On précipite dans un mélange eau/glace, puis on extrait à l'éther éthylique et on sèche sur sulfate de magnésium.
On concentre la solution obtenue et on précipite dans un mélange méthanol/glace (90/10) dans un rapport composé/précipitant de 1/5.
On obtient 23 g de composé, se présentant sous forme de poudre, soit un rendement de 85%.

La caractérisation est effectuée par RMN/ GPC ou HPLC. Le composé obtenu présente des valeurs conformes à celles attendues.

### Exemple 2 : Préparation d'un polymère-étoile à 8 branches dont chaque branche est un copolymère bloc

### 1/Première étape : préparation d'un polymère-étoile à 8 branches de polyacrylate de tertiobutyle

Les réactifs employés sont les suivants:
- monomère 1: acrylate de tertiobutyle (Tg = 50°C) 100 g
- monomère 2 : acrylate d'isobutyle (Tg = -20°C) 20 g
- initiateur (préparé selon l'exemple 1) (correspondant à 4.10⁻³ mole de RBr) 1,19 g
- CuBr (correspondant à 4.10⁻³ mol) 0, 57 g
- Bipyridine (correspondant à 8.10⁻³ mol) 1,25 g

Les monomères sont préalablement distillés.
Dans un réacteur hermétique, flambé et comportant une arrivée d'azote, on mélange les réactifs sauf les monomères, puis on ajoute le monomère 1.
On chauffe, sous azote, à 120°C environ puis on laisse réagir à 120°C pendant 4 heures, en coupant l'arrivée d'azote.

### 2/Deuxième étape : formation du deuxième bloc à l'extrémité de chaque branche

On ajoute alors le monomère 2 et on laisse à nouveau réagir à 120°C, pendant 4 heures.

Après réaction, on laisse refroidir le mélange réactionnel; on obtient une solution visqueuse verte que l'on dissout dans le dichlorométhane. On passe la solution de polymère sur alumine neutre et on précipite la solution limpide obtenue dans un mélange méthanol/eau (80/20) dans un rapport polymère/précipitant de 1/5.

On obtient 115 g de polymère se présentant sous forme de produit visqueux, soit un rendement de 96%.
Ce polymère est un polymère-étoiles à 8 branches de polyacrylate d'isobutyle, dont chaque branche est un copolymère bloc : le calix(polyacrylate de tertiobutyle-bloc-polyacrylate d'isobutyle).

La caractérisation est effectuée par GPC : THF équivalent polystyrène linéaire, détection diffusion de lumière : 350 000 g/mol (masse théorique : 240 000 environ); indice de polydispersité : 1,6.
Le polymère obtenu présente des valeurs conformes à celles attendues.
Le polymère est soluble dans l'éthanol.
Dureté : 50 s.

### Exemple 3 Vernis à ongles

On prépare une composition de vernis à ongles comprenant :
- polymère obtenu selon l'exemple 2 25%
- pigments qs
- solvants (acétate d'éthyle/ acétate de butyle (90/10)) qsp 100%

Le polymère est solubilisé dans le mélange de solvants. On obtient une composition de vernis qui s'étale aisément et permet l'obtention d'un film brillant et de dureté adéquate.

## Revendications

1. Composition cosmétique de soin ou de maquillage des ongles, naturels ou artificiels, comprenant, dans un milieu cosmétiquement acceptable comprenant au moins un constituant choisi parmi l'eau; un ou plusieurs solvants cosmétiquement acceptables tels que les alcools en C₁-C₄, les éthers liquides à 25°C, les cétones liquides à 25°C, les esters d'acide carboxylique inférieurs en C₁-C₈ ; les alcanes liquides à 25°C; les composés cycliques aromatiques liquides à 25°C; les aldéhydes liquides à 25°C; les huiles de silicone, minérales, d'origine animale ou végétale, de synthèse ou fluorées, volatiles ou non; les colorants hydrosolubles; les pigments; les nacres, les laques; les épaississants, les gélifiants; les tensioactifs; les plastifiants; les polymères hydrophiles ou hydrosolubles; les résines alkydes, acryliques et/ou vinyliques, les polyuréthannes et les polyesters, les celluloses et dérivés cellulosiques telles que la nitrocellulose; les résines résultant de la condensation de formaldéhyde avec une arylsulfonamide; des polymères filmogènes en dispersion aqueuse tels que les polyuréthannes, les polyesters-polyuréthannes, les polyéther-polyuréthannes, les polymères radicalaires notamment de type acrylique, acrylique styrène et/ou vinylique, les polyesters, les résines alkydes; leurs mélanges; un agent auxiliaire de filmification, tel qu'un agent plastifiant et/ou un agent de coalescence;
au moins un polymère de structure en "étoiles" susceptible d'être obtenu par polymérisation radicalaire par transfert d'atomes :
- d'un ou plusieurs monomères polymérisables radicalairement, choisis parmi :
(i) les esters acryliques ou méthacryliques obtenus à partir d'alcools aliphatiques, linéaires, ramifiés, cycliques et/ou d'alcools aromatiques, en C₁-C₂₀ tel que le (méth)acrylate de méthyle, le (méth)acrylate d'éthyle, le (méth)acrylate de propyle, le (méth)acrylate de butyle, le (méth)acrylate d'isobutyle, le (méth)acrylate de tertio-butyle ;
(ii) les (méth)acrylates d'hydroxyalkyle en C₁-C₄ tels que le (méth)acrylate de 2-hydroxyéthyle ou le (méth)acrylate de 2-hydroxypropyle;
(iii) les (méth)acrylates d'éthylèneglycol, de diéthylèneglycol, de polyéthylèneglycol, à extrémité hydroxyle ou éther;
(iv) les esters vinyliques, allyliques ou méthallyliques obtenus à partir d'alcools aliphatiques, linéaires ou ramifiés en C₁-C₁₀ ou cycliques en C₁-C₆ et/ou d'alcools aromatiques, en C₁-C₆, tels que l'acétate de vinyle, le propionate de vinyle, le benzoate de vinyle, le tertio-butyl benzoate de vinyle ;
(v) la N-vinylpyrrolidone; la vinylcaprolactame; les vinyl N-alkylpyrroles ayant 1 à 6 atomes de carbone; les vinyl-oxazoles; les vinyl-thiazoles; les vinylpyrrimidines; les vinylimidazoles; les vinyl cétones;
(vi) les amides (méth)acryliques obtenues à partir d'amines aliphatiques, linéaires, ramifiés, cycliques et/ou d'amines aromatiques, en C₁-C₂₀ telles que le tertiobutylacrylamide; les (méth)acrylamides tels que l'acrylamide, le méthacrylamide, les dialkyl(C₁-C₄) (méth)acrylamides ;
(vii) les oléfines tels que l'éthylène, le propylène, le styrène ou le styrène substitué;
(viii) les monomères acryliques ou vinyliques fluorés ou perfluorés, notamment les esters (méth)acryliques à motifs perfluoroalkyle;
(ix) les monomères comportant une fonction amine sous forme libre ou bien partiellement ou totalement neutralisée ou bien partiellement ou totalement quaternisée tels que le (méth)acrylate de diméthylaminoéthyle, le diméthylaminoéthyl méthacrylamide, la vinylamine, la vinylpyridine, le chlorure de diallyidiméthylammonium;
(x) les carboxybétaïnes ou les sulfobétaïnes obtenues par quaternisation partielle ou totale de monomères à insaturation éthylénique comportant une fonction amine par des sels de sodium d'acide carboxylique à halogénure mobile (chloroacétate de sodium par exemple) ou par des sulfones cycliques (propane sulfone);
(xi) les (méth)acrylates ou (méth)acrylamides siliconés, notamment les esters(méth)acryliques à motifs siloxane;
(xii) leurs mélanges;
en présence
- d'un initiateur ayant au moins deux atomes et/ou groupes radicalairement transférables par polymérisation,
- d'un composé comprenant un métal de transition, susceptible de participer à une étape de réduction avec l'initiateur et une chaîne polymérique "dormante", choisi parmi ceux de formule Mⁿ⁺X'ₙ, dans laquelle :
- M est choisi parmi Cu, Au, Ag, Hg, Ni, Pd, Pt, Rh, Co, Ir, Fe, Ru, Os, Re, Mn, Cr, Mo, W, V, Nb, Ta et Zn, et
- X' représente un halogène (brome ou chlore notamment), OH, (O)_{1/2}, un radical alcoxy ayant 1-6 atomes de carbone, (SO₄)_{1/2}, (PO₄)_{1/3}, (HPO₄)_{1/2}, (H₂PO₄), un radical triflate, hexafluorophosphate, methanesulfonate, arylsulfonate, SeR, CN, NC, SCN, CNS, OCN, CNO, N₃ et R'CO₂, dans lequel R représente un radical aryle ou alkyle, linéaire ou ramifié, ayant 1-20, de préférence 1-10 atomes de carbone, et R' représente H ou un radical alkyle, linéaire ou ramifié, ayant 1-6 atomes de carbone, ou un radical aryle, éventuellement substitué par un ou plusieurs atomes d'halogènes notamment de fluor et/ou de chlore;
- n est la charge du métal;
et
- d'un ligand choisi parmi :
(i) les composés comprenant au moins un atome d'azote (N), d'oxygène (O), de phosphore (P) et/ou de soufre (S), susceptibles de se coordonner par une liaison σ audit composé comprenant un métal de transition;
(ii) les composés comprenant au moins deux atomes de carbone susceptibles de se coordonner par une liaison π audit composé comprenant un métal de transition;
(iii) les composés comprenant au moins un atome de carbone susceptibles de se coordonner par une liaison σ audit composé comprenant un métal de transition, mais qui ne forment pas de liaison carbone-carbone avec le monomère lors de la polymérisation;
(iv) les composés susceptibles de se coordonner par une liaison µ ou η audit composé comprenant un métal de transition;
ledit polymère comprenant un ou plusieurs monomères Mi dont l'homopolymère correspondant présente une Tg supérieure ou égale à 0°C, de préférence supérieure ou égale à 5°C, et encore mieux supérieure ou égale à 10°C; ce ou ces monomères Mi étant présents, dans le polymère final, en une quantité de 55-98% en poids, de préférence en une quantité de 75 à 95% en poids, et encore mieux en une quantité de 80-90% en poids, par rapport au poids total de monomères, et
un ou plusieurs monomères Mj dont l'homopolymère correspondant présente une Tg inférieure ou égale à 0°C, de préférence inférieure ou égale à -5°C, et encore mieux inférieure ou égale à -10°C; ce ou ces monomères Mj étant présents, dans le polymère final, en une quantité de 2-45% en poids, de préférence en une quantité de 5-25% en poids, et encore mieux en une quantité de 10-20% en poids, par rapport au poids total de monomères.

2. Composition selon la revendication 1, dans laquelle les monomères sont choisis parmi :
- les esters (méth)acryliques obtenus à partir d'alcools aliphatiques, linéaires ou ramifiés, de préférence en C₁-C₂₀;
- les esters (méth)acryliques en C₁-C₂₀ à motifs perfluoroalkyle;
- les esters(méth)acryliques en C₁-C₂₀ à motifs siloxane;
- les amides (méth)acryliques obtenues à partir d'amines aliphatiques, linéaires, ramifiés, cycliques et/ou d'amines aromatiques, de préférence en C₁-C₂₀ telles que le tertiobutylacrylamide; les (méth)acrylamides tels que l'acrylamide, le méthacrylamide, les dialkyl(C₁-C₄) (méth)acrylamides ;
- les esters vinyliques, allyliques ou méthallyliques obtenus à partir d'alcools aliphatiques, linéaires ou ramifiés en C₁-C₁₀ ou cycliques en C₁-C₆ ;
- la vinylcaprolactame ;
- le styrène éventuellement substitué;
- leurs mélanges.

3. Composition selon l'une des revendications précédentes, dans laquelle l'initiateur est choisi parmi les composés de formule :
- R¹¹ₓR¹²_{y}R¹³_{z}C-(RX)ₜ dans laquelle x, y et z représentent un entier allant de 0 à 4, t un entier allant de 1 à 4, et x+y+z= 4-t;
- R¹³ₓC₆-(RX)_{y} (cycle à 6 carbones, saturé) dans laquelle x représente un entier allant de 7 à 11, y représente un entier allant de 1 à 5, et x+y= 12 ;
- R¹³ₓC₆-(RX)_{y} (cycle à 6 carbones, insaturé) dans laquelle x représente un entier allant de 0 à 5, y représente un entier allant de 1 à 6, et x+y= 6;
- -[-(R¹¹)(R¹²)(R¹³)C-(RX)-]ₙ dans laquelle n est supérieur ou égal à 1; cyclique ou linéaire;
- -[-(R¹²)ₓ C₆ (RX)_{y}- R¹¹-]ₙ dans laquelle x représente un entier allant de 0 à 6, y représente un entier allant de 1 à 6, et n est supérieur ou égal à 1, avec x+y=4 ou 6; cyclique ou linéaire;
- -[-(R¹²)ₓ C₆ (RX)_{y}- R¹¹-]ₙ dans laquelle x représente un entier allant de 0 à 12, y représente un entier allant de 1 à 12, et n est supérieur ou égal à 1, avec x+y=10 ou 12; cyclique ou linéaire;
- -[OSi (R¹¹)ₓ(RX)_{y}]ₙ, cyclique ou linéaire, dans laquelle x et y représentent un entier allant de 0 à 2, et n est supérieur ou égal à 1, avec x+y=2;
- R¹¹N-X₂
- (R¹¹)ₓP(O)_{y}-X₃₋ₓ dans laquelle x et y représentent des entiers allant de 0 à 2, et x+y = 5;
- (R¹¹O)ₓP(O)_{y}-X₃₋ₓ dans laquelle x et y représentent des entiers allant de 0 à 2, et x+y = 5;
- -[(R¹¹)ₜN_{z}P(O)ₓ(O-RX)_{y}-]ₙ, cyclique ou linéaire, dans laquelle x représente un entier allant de 0 à 4, y représente un entier allant de 1 à 5, z représente un entier allant de 0 à 2 et t représente un entier allant de 0 à 3, et n est supérieur ou égal à 1;
dans lesquelles :
- R, R¹¹, R¹² et R¹³ représentent, indépendamment l'un de l'autre, un atome d'hydrogène ou d'halogène; un radical alkyle, linéaire ou ramifié, ayant 1-20, de préférence 1-10 et plus préférentiellement 1-6 atomes de carbone; un radical cycloalkyle ayant 3-8 atomes de carbone; un radical -C(=Y)R⁵, -C(=Y)NR⁶R⁷ ou -R⁸₃Si; -COCl, -OH, -CN, un radical alkényle ou alkynyle ayant 2-20, de préférence 2-6, atomes de carbone; un radical oxiranyle, glycidyle, alkylène ou alkénylène substitué avec un oxiranyle ou un glycidyle; un radical aryle, heterocyclyle, aralkyle, aralkenyle; un radical alkyle ayant 1-6 atomes de carbone dans lequel tout ou partie des atomes d'hydrogène sont substitués soit par des atomes d'halogènes tels que fluor, chlore ou brome, soit par un groupe alcoxy ayant 1-4 atomes de carbone ou par un radical aryle, heterocyclyle, -C(=Y)R⁵, -C(=Y)NR⁶R⁷, oxiranyle, glycidyle;
- X représente un atome d'halogène tel que Cl, Br, I, ou un radical -OR', -SR, -SeR, -OC(=O)R', -OP(=O)R', -OP(=O)(OR')₂, -OP(=O)OR', -O-NR'₂, -S-C(=S)NR'₂,-CN, -NC, -SCN, -CNS, -OCN, -CNO et -N₃, dans lequel R' représente un radical alkyle ayant 1-20 atomes de carbone éventuellement substitué par un ou plusieurs atomes d'halogènes notamment de fluor et/ou de chlore; et R représente un radical aryle ou alkyle, linéaire ou ramifié, ayant 1-20, de préférence 1-10, atomes de carbone; le groupement -NR'₂ pouvant en outre représenter un groupement cyclique, les deux groupes R' étant joints de manière à former un hétérocycle à 5, 6 ou 7 membres;
- Y représente O, S ou NR⁸ (de préférence O),
- R⁵ représente un radical alkyle, alkylthio, alcoxy, linéaire ou ramifié, ayant 1-20 atomes de carbone; un radical OH; un radical OM' avec M' = métal alcalin; un radical aryloxy ou un radical heterocyclyloxy;
- R⁶ et R⁷ représentent, indépendamment l'un de l'autre, H ou un radical alkyle, linéaire ou ramifié, ayant 1-20 atomes de carbone; étant donné que R⁶ et R⁷ peuvent être joints pour former un groupe alkylène ayant 2-7, de préférence 2-5, atomes de carbone;
- R⁸ représente H; un radical alkyle, linéaire ou ramifié, ayant 1-20 atomes de carbone ou un radical aryle.

4. Composition selon l'une des revendications précédentes, dans laquelle l'initiateur est choisi parmi :
- l'octa-2-isobutyrylbromide-octatertiobutyl-calix(8)arène,
- l'octa-2-propionylbromide -octatertiobutyl-calix(8)arène, et
- l'hexakis α-bromométhylbenzène.

5. Composition selon l'une des revendications précédentes, dans laquelle le ligand est choisi parmi :
(i) les composés de formule :
R⁹-Z-(R¹⁴-Z)ₘ-R¹⁰ ou R²⁰R²¹C[C(=Y)R⁵]
dans lesquelles :
- R⁹ et R¹⁰ sont, indépendamment l'un de l'autre, un atome d'hydrogène; un radical alkyle, linéaire ou ramifié, ayant 1-20, de préférence 1-10 atomes de carbone; un radical aryle; un radical hétérocyclyle; un radical alkyle ayant 1-6 atomes de carbone substitué avec un radical alcoxy ayant 1-6 atomes de carbone ou un radical dialkylamino ayant 1-4 atomes de carbone ou un radical -C(=Y)R⁵ ou -C(=Y)NR⁶R⁷ et/ou YC(=Y)R⁸, R⁵ à R⁸ et Y ayant les définitions données en revendication 3; étant donné que R⁹ et R¹⁰ peuvent être joints de manière à former un cycle, saturé ou insaturé;
- R¹⁴ représente, indépendamment les uns des autres, un groupe divalent choisi parmi les alcanediyles ayant 2-4 atomes de carbone; les alkénylènes ayant 2-4 atomes de carbone; les cycloalcanediyles ayant 3-8 atomes de carbone; les cycloalkènediyles ayant 3-8 atomes de carbone; les arènediyles et les hétérocyclylènes;
- Z représente O, S, NR¹⁵ ou PR¹⁵ avec R¹⁵ représentant H; un radical alkyle, linéaire ou ramifié, ayant 1-20 atomes de carbone; un radical aryle; un radical heterocyclyle; un radical alkyle ayant 1-6 atomes de carbone substitué avec un radical alcoxy ayant 1-6 atomes de carbone ou un radical dialkylamino ayant 1-4 atomes de carbone ou un radical -C(=Y)R⁵ ou -C(=Y)NR⁶R⁷ et/ou YC(=Y)R⁸, R⁵ à R⁸ et Y ayant les définitions données dans la revendication 3;
- m est compris entre 0 et 6;
- R²⁰ et R²¹ sont, indépendamment l'un de l'autre, un atome d'hydrogène; un atome d'halogène; un radical alkyle, linéaire ou ramifié, ayant 1-20, de préférence 1-10 atomes de carbone; un radical aryle; un radical hétérocyclyle; étant donné que R²⁰ et R²¹ peuvent être joints de manière à former un cycle, saturé ou insaturé; étant donné que chaque radical peut en outre être substitué avec un radical alkyle ayant 1-6 atomes de carbone, un radical alcoxy ayant 1-6 atomes de carbone ou un radical aryle;
(ii) le monoxyde de carbone; les porphyrines et les porphycènes, éventuellement substitués; l'éthylènediamine et le propylènediamine, éventuellement substitués; les multiamines avec amines tertiaires telles que le pentaméthyldiéthylènetriamine; les aminoalcools tels que l'aminoéthanol et l'aminopropanol, éventuellement substitués; les glycols tels que l'éthylèneglycol ou le propylèneglycol, éventuellement substitués; les arènes tels que le benzène, éventuellement substitués; le cyclopentadiène, éventuellement substitué; les pyridines et bipyridines, éventuellement substituées; I'acétonitrile; la 1,10-phénanthroline; les cryptands et les éthers-couronnes; la spartéine.

6. Composition selon l'une des revendications précédentes, dans laquelle le polymère de structure "en étoiles" est présent en une quantité comprise entre 1-60% en poids de matière sèche, par rapport au poids total de la composition, de préférence entre 1-50% en poids et préférentiellement entre 5-40% en poids.

7. Composition selon l'une des revendications précédentes, dans laquelle le polymère de structure "en étoiles" permet l'obtention d'un film ayant une dureté de surface inférieure à 110, de préférence comprise entre 1 et 70 et encore mieux comprise entre 5 et 55.

8. Composition selon l'une des revendications précédentes, dans laquelle le polymère de structure "en étoiles" est présent dans le milieu sous forme dissoute ou en dispersion, dans une phase aqueuse, organique, hydroorganique notamment alcoolique ou hydroalcoolique.

9. Composition selon l'une des revendications précédentes, se présentant sous forme de solutions ou de dispersions aqueuses ou en milieu solvant.

10. Composition selon l'une des revendications précédentes, se présentant sous la forme d'une base de soin pour les ongles ou d'un vernis à ongles.

11. Procédé de traitement cosmétique des ongles, **caractérisé en ce qu'**il consiste à appliquer sur ces derniers une composition cosmétique selon l'une des revendications 1 à 10.

12. Utilisation d'au moins un polymère tel que défini dans la revendication 1, dans une composition cosmétique permettant l'obtention d'un film présentant une bonne résistance à l'écaillage; aux chocs, aux frottements, aux rayures et/ou aux pressions.

## Claims

1. Cosmetic care or makeup composition for natural or artificial nails, comprising, in a cosmetically acceptable medium comprising at least one constituent chosen from water; one or more cosmetically acceptable solvents such as C₁-C₄ alcohols, ethers that are liquid at 25°C, ketones that are liquid at 25°C, C₁-C₈ lower carboxylic acid esters; alkanes that are liquid at 25°C; cyclic aromatic compounds that are liquid at 25°C; aldehydes that are liquid at 25°C; silicone oils, mineral oils, animal oils, plant oils, synthetic oils or fluoro oils, which may be volatile or non-volatile; water-soluble colorants; pigments; nacres, lakes; thickeners, gelling agents; surfactants; plasticizers; hydrophilic or water-soluble polymers; alkyd resins,. acrylic resins and/or vinyl resins, polyurethanes and polyesters, celluloses and cellulose derivatives such as nitrocellulose; resins resulting from the condensation of formaldehyde with an arylsulphonamide; film-forming polymers in aqueous dispersion such as polyurethanes, polyurethane polyesters, polyurethane polyethers, free-radical polymers especially of acrylic, styreneacrylic and/or vinyl type, polyesters and alkyd resins; mixtures thereof; an auxiliary film-forming agent such as a plasticizer and/or a coalescer;
at least one "starburst" structural polymer that may be obtained by atom-transfer radical polymerization:
- of one or more free-radical-polymerizable monomers chosen from:
(i) acrylic or methacrylic esters obtained from C₁-C₂₀ linear, branched or cyclic aliphatic alcohols and/or from C₁-C₂₀ aromatic alcohols, such as methyl (meth)acrylate, ethyl (meth)acrylate, propyl (meth)acrylate, butyl (meth)acrylate, isobutyl (meth)acrylate or tert-butyl (meth)acrylate;
(ii) C₁-C₄ hydroxyalkyl (meth)acrylates such as 2-hydroxyethyl (meth)acrylate or 2-hydroxypropyl (meth)acrylate;
(iii) ethylene glycol, diethylene glycol or polyethylene glycol (meth)acrylates, with a hydroxyl or ether end;
(iv) vinyl, allylic or methallylic esters obtained from linear or branched C₁-C₁₀ aliphatic alcohols or cyclic C₁-C₆ aliphatic alcohols, and/or from C₁-C₆ aromatic alcohols, such as vinyl acetate, vinylpropionate, vinylbenzoate or vinyl tert-butylbenzoate;
(v) N-vinylpyrrolidone; vinylcaprolactam; vinyl N-alkylpyrroles containing 1 to 6 carbon atoms; vinyloxazoles; vinylthiazoles; vinylpyrimidines; vinylimidazoles; vinyl ketones;
(vi) (meth)acrylic amides obtained from linear, branched or cyclic C₁-C₂₀ aliphatic amines and/or from C₁-C₂₀ aromatic amines, such as tert-butylacrylamide; (meth)acrylamides such as acrylamide, methacrylamide and di(C₁-C₄)alkyl(meth) acrylamides;
(vii) olefins such as ethylene, propylene, styrene or substituted styrene;
(viii) acrylic or vinyl monomers, which may be fluorinated or perfluorinated, especially (meth)acrylic esters containing perfluoroalkyl units;
(ix) monomers comprising an amine function in free form or in partially or totally neutralized form or in partially or totally quaternized form, such as dimethylaminoethyl (meth)acrylate, dimethylaminoethyl-methacrylamide, vinylamine, vinylpyridine or diallyldimethylammonium chloride;
(x) carboxybetaines or sulphobetaines obtained by partial or total quaternization of ethylenically unsaturated monomers comprising an amine function, with carboxylic acid sodium salts containing a labile halide (for example sodium chloroacetate) or with cyclic sulphones (propane sulphone);
(xi) silicone (meth)acrylates or (meth)acrylamides, especially (meth)acrylic esters containing siloxane units;
(xii) mixtures thereof;
in the presence of
- an initiator containing at least two atoms and/or groups that can be transferred by free-radical polymerization,
- a compound comprising a transition metal, capable of participating in a reduction step with the initiator and a "dormant" polymer chain, chosen from those of formula Mⁿ⁺X'ₙ, in which:
- M is chosen from Cu, Au, Ag, Hg, Ni, Pd, Pt, Rh, Co, Ir, Fe, Ru, Os, Re, Mn, Cr, Mo, W, V, Nb, Ta and Zn, and
- X' represents a halogen (especially bromine or chlorine), OH, (O)_{1/2}, an alkoxy radical containing 1-6 carbon atoms, (SO₄)_{1/2}, (PO₄)_{1/3}, (HPO₄)_{1/2}, (H₂PO₄), a triflate, hexafluorophosphate, methanesulphonate, arylsulphonate, SeR, CN, NC, SCN, CNS, OCN, CNO, N₃ or R'CO₂ radical, in which R represents a linear or branched aryl or alkyl radical containing 1-20 and preferably 1-10 carbon atoms, and R' represents H or a linear or branched alkyl radical containing 1-6 carbon atoms, or an aryl radical optionally substituted with one or more halogen atoms, especially fluorine and/or chlorine;
- n is the charge of the metal;
and
- a ligand chosen from:
(i) compounds comprising at least one nitrogen (N), oxygen (O), phosphorus (P) and/or sulphur (S) atom, which are capable of coordinating via a σ bond to the said compound comprising a transition metal;
(ii) compounds comprising at least two carbon atoms, which are capable of coordinating via a π bond to the said compound comprising a transition metal;
(iii) compounds comprising at least one carbon atom, which are capable.of coordinating via a σ bond to the said compound comprising a transition metal, but which do not form a carbon-carbon bond with the monomer during the polymerization;
(iv) compounds capable of coordinating via a µ or η bond to the said compound comprising a transition metal;
the said polymer comprising one or more monomers Mi whose corresponding homopolymer has a Tg of greater than or equal to 0°C, preferably greater than or equal to 5°C and better still greater than or equal to 10°C; this or these monomer(s) Mi being present in the final polymer in an amount of 55-98% by weight, preferably in an amount of from 75% to 95% by weight and better still in an amount of 80-90% by weight, relative to the total weight of monomers, and
one or more monomers Mj whose corresponding homopolymer has a Tg of less than or equal to 0°C, preferably less than or equal to -5°C and better still less than or equal to -10°C; this or these monomer(s) Mj being present in the final polymer in an amount of 2-45% by weight, preferably in an amount of 5-25% by weight and better still in an amount of 10-20% by weight, relative to the total weight of monomers.

2. Composition according to Claim 1, in which the monomers are chosen from:
- (meth)acrylic esters obtained from linear or branched aliphatic alcohols, which are preferably C₁-C₂₀;
- C₁-C₂₀ (meth)acrylic esters containing perfluoroalkyl units;
- C₁-C₂₀ (meth)acrylic esters containing siloxane units;
- (meth)acrylic amides obtained from linear, branched or cyclic preferably C₁-C₂₀ aliphatic amines and/or from preferably C₁-C₂₀ aromatic amines, such as tert-butylacrylamide; (meth)acrylamides such as acrylamide, methacrylamide and di(C₁-C₄)alkyl(meth)acrylamides;
- vinyl, allylic or methallylic esters obtained from linear or branched C₁-C₁₀ aliphatic alcohols or cyclic C₁-C₆ aliphatic alcohols;
- vinylcaprolactam;
- optionally substituted styrene;
- mixtures thereof.

3. Composition according to either of the preceding claims, in which the initiator is chosen from the compounds of formula:
- R¹¹ₓR¹²_{y}R¹³_{z}C-(RX)ₜ in which x, y and z represent an integer ranging from 0 to 4, t represents an integer ranging from 1 to 4, and x+y+z = 4-t;
- R¹³ₓC₆-(RX)_{y} (saturated 6-carbon ring) in which x represents an integer ranging from 7 to 11, y represents an integer ranging from 1 to 5, and x+y = 12;
- R¹³ₓC₆-(RX)_{y} (unsaturated 6-carbon ring) in which x represents an integer ranging from 0 to 5, y represents an integer ranging from 1 to 6, and x+y =6;
- -[-(R¹¹) (R¹²) (R¹³)C-(RX)-]ₙ in which n is greater than or equal to 1; cyclic or linear;
- -[-(R¹²)ₓC₆(RX)_{y}-R¹¹-]ₙ in which x represents an integer ranging from 0 to 6, y represents an integer ranging from 1 to 6, and n is greater than or equal to 1, with x+y = 4 or 6; cyclic or linear;
- -[-(R¹²)ₓC₆(RX)_{y}-R¹¹-]ₙ in which x represents an integer ranging from 0 to 12, y represents an integer ranging from 1 to 12 and n is greater than or equal to 1, with x+y = 10 or 12; cyclic or linear;
- -[OSi(R¹¹)ₓ(RX)_{y}]ₙ, cyclic or linear, in which x and y represent an integer ranging from 0 to 2, and n is greater than or equal to 1, with x+y = 2;
- R¹¹N-X₂
- (R¹¹)ₓP(O)_{y}-X₃₋ₓ in which x and y represent integers ranging from 0 to 2, and x+y =5;
- (R¹¹O)ₓP(O)_{y}-X₃₋ₓ in which x and y represent integers ranging from 0 to 2, and x+y = 5;
- -[(R¹¹)ₜN_{z}P(O)ₓ(O-RX)_{y}-]ₙ, cyclic or linear, in which x represents an integer ranging from 0 to 4, y represents an integer ranging from 1 to 5, z represents an integer ranging from 0 to 2 and t represents an integer ranging from 0 to 3, and n is greater than or equal to 1;
in which:
- R, R¹¹, R¹² and R¹³ represent, independently of each other, a hydrogen or halogen atom; a linear or branched alkyl radical containing 1-20, preferably 1-10 and more preferably 1-6 carbon atoms; a cycloalkyl radical containing 3-8 carbon atoms; a radical -C(=Y)R⁵, -C(=Y)NR⁶R⁷ or -R⁸₃Si; -COCl, -OH, -CN, an alkenyl or alkynyl radical containing 2-20 and preferably 2-6 carbon atoms; an oxiranyl, glycidyl, alkylene or alkenylene radical substituted with an oxiranyl or a glycidyl; an aryl, heterocyclyl, aralkyl or aralkenyl radical; an alkyl radical containing 1-6 carbon atoms in which all or some of the hydrogen atoms are substituted either with halogen atoms such as fluorine, chlorine or bromine, or with an alkoxy group containing 1-4 carbon atoms or with an aryl, heterocyclyl, -C(=Y)R⁵, -C(=Y)NR⁶R⁷, oxiranyl or glycidyl radical;
- X represents a halogen atom such as Cl, Br or I, or a radical -OR', -SR, -SeR, -OC(=O)R', -OP(=O)R', -OP(=O)(OR')₂, -OP(=O)OR', -O-NR'₂, -S-C(=S)NR'₂, -CN, -NC, -SCN, -CNS, -OCN, -CNO and -N₃, in which R' represents an alkyl radical containing 1-20 carbon atoms, optionally substituted with one or more halogen atoms, especially fluorine and/or chlorine; and R represents a linear or branched alkyl or aryl radical containing 1-20 and preferably 1-10 carbon atoms; the group -NR'₂ also possibly representing a cyclic group, the two groups R' being joined together so as to form a 5-, 6- or 7-membered heterocycle;
- Y represents O, S or NR⁸ (preferably O),
- R⁵ represents a linear or branched alkyl, alkylthio or alkoxy radical containing 1-20 carbon atoms; an OH radical; a radical OM' in which M' = alkali metal; an aryloxy radical or a heterocyclyloxy radical;
- R⁶ and R⁷ represent, independently of each other, H or a linear or branched alkyl radical containing 1-20 carbon atoms; given that R⁶ and R⁷ may be joined together to form an alkylene group containing 2-7 and preferably 2-5 carbon atoms;
- R⁸ represents H; a linear or branched alkyl radical containing 1-20 carbon atoms, or an aryl radical.

4. Composition according to one of the preceding claims, in which the initiator is chosen from:
- octa-2-isobutyrylbromideocta-tert-butylcalix(8)arene,
- octa-2-propionylbromideocta-tert-butylcalix(8)arene, and
- hexakis(α-bromomethyl)benzene.

5. Composition according to one of the preceding claims, in which the ligand is chosen from:
(i) the compounds of formula: R⁹-Z-(R¹⁴-Z)ₘ-R¹⁰ or R²⁰R²¹C[C(=Y)R⁵]
in which:
- R⁹ and R¹⁰ are, independently of each other, a hydrogen atom; a linear or branched alkyl radical containing 1-20 and preferably 1-10 carbon atoms; an aryl radical; a heterocyclyl radical; an alkyl radical containing 1-6 carbon atoms, substituted with an alkoxy radical containing 1-6 carbon atoms or a dialkylamino radical containing 1-4 carbon atoms or a radical-C(=Y)R⁵ or -C(=Y)NR⁶R⁷ and/or YC(=Y)R⁸, R⁵ to R⁸ and Y having the definitions given in Claim 3; it being given that R⁹ and R¹⁰ may be joined together so as to form a saturated or unsaturated ring;
- R¹⁴ represents, independently of each other, a divalent group chosen from alkanediyls containing 2-4 carbon atoms; alkenylenes containing 2-4 carbon atoms; cycloalkanediyls containing 3-8 carbon atoms; cycloalkenediyls containing 3-8 carbon atoms; arenediyls and heterocyclylenes;
- Z represents O, S, NR¹⁵ or PR¹⁵, with R¹⁵ representing H; a linear or branched alkyl radical containing 1-20 carbon atoms; an aryl radical; a heterocyclyl radical; an alkyl radical containing 1-6 carbon atoms, substituted with an alkoxy radical containing 1-6 carbon atoms or a dialkylamino radical containing 1-4 carbon atoms or a radical -C(=Y)R⁵ or -C(=Y)NR⁶R⁷ and/or YC(=Y)R⁸, R⁵ to R⁸ and Y having the definitions given in Claim 3;
- m is between 0 and 6;
- R²⁰ and R²¹ are, independently of each other, a hydrogen atom; a halogen atom; a linear or branched alkyl radical containing 1-20 and preferably 1-10 carbon atoms; an aryl radical; a heterocyclyl radical; given that R²⁰ and R²¹ may be joined together so as to form a saturated or unsaturated ring; given that each radical may also be substituted with an alkyl radical containing 1-6 carbon atoms; an alkoxy radical containing 1-6 carbon atoms or an aryl radical;
(ii) carbon monoxide; optionally substituted porphyrines and porphycenes; optionally substituted ethylenediamine and propylenediamine; polyamines with tertiary amines such as pentamethyldiethylenetriamine; optionally substituted amino alcohols, such as aminoethanol and aminopropanol; optionally substituted glycols, such as ethylene glycol or propylene glycol; optionally substituted arenes, such as benzene; optionally substituted cyclopentadiene; optionally substituted pyridines and bipyridines; acetonitrile; 1,10-phenanthroline; cryptands and crown ethers; sparteine.

6. Composition according to one of the preceding claims, in which the polymer of "starburst" structure is present in an amount of between 1-60% by weight of solids, preferably between 1-50% by weight and preferentially between 5-40% by weight, relative to the total weight of the composition.

7. Composition according to one of the preceding claims, in which the polymer of "starburst" structure allows the production of a film that has a surface hardness of less than 110, preferably between 1 and 70 and better still between 5 and 55.

8. Composition according to one of the preceding claims, in which the polymer of "starburst" structure is present in the medium in dissolved or dispersed form, in an aqueous, organic or aqueous-organic phase, especially an alcoholic or aqueous-alcoholic phase.

9. Composition according to one of the preceding claims, which is in the form, of aqueous solutions or dispersions or is in a solvent medium.

10. Composition according to one of the preceding claims, which is in the form of a nailcare base or a nail varnish.

11. Cosmetic process for treating the nails, **characterized in that** it consists in applying to the nails a cosmetic composition according to one of Claims 1 to 10.

12. Use of at least one polymer as defined in Claim 1, in a cosmetic composition for obtaining a film that shows good resistance to flaking, to impacts, to rubbing, to scratching and/or to pressure.

## Patentansprüche

1. Kosmetische Zusammensetzung zur Pflege oder zum Schminken von natürlichen oder künstlichen Nägeln, die in einem kosmetisch akzeptablen Medium mindestens einen Bestandteil, der unter den folgenden Verbindungen ausgewählt ist: Wasser; einem oder mehreren kosmetisch akzeptablen Lösungsmitteln, wie C₁₋₄-Alkoholen, bei 25 °C flüssigen Ethern, bei 25 °C flüssigen Ketonen, Estern von niederen Carbonsäuren mit 1 bis 8 Kohlenstoffatomen; bei 25 °C flüssigen Alkanen; bei 25 °C flüssigen cyclischen aromatischen Verbindungen; bei 25 °C flüssigen Aldehyden; Siliconölen, Mineralölen, Ölen tierischer Herkunft, Ölen pflanzlicher Herkunft, synthetischen Ölen oder fluorierten Ölen, die flüchtig oder nicht flüchtig sind; wasserlöslichen Farbstoffen; Pigmenten; Perlglanzpigmenten, Lacken; Verdickungsmitteln; Gelbildnern; grenzflächenaktiven Stoffen; Weichmachern; hydrophilen oder wasserlöslichen Polymeren; Alkydharzen, Acrylharzen und/oder Vinylharzen; Polyurethanen, Polyestern; Cellulosen und Cellulosederivaten wie Nitrocellulose; Harzen, die bei der Kondensation von Formaldehyd und einem Arylsulfonamid gebildet werden; filmbildenden Polymeren in wässriger Dispersion, z.B. Polyurethanen, Polyester-Polyurethanen, Polyether-Polyurethanen, durch radikalische Polymerisation hergestellten Polymeren insbesondere vom Acryltyp, Acryl/Styroltyp und/oder Vinyltyp, Polyestern, Alkydharzen; und deren Gemischen; Hilfsstoffen für die Filmbildung wie Weichmachern und/oder Koaleszenzmitteln; und mindestens ein Polymer mit "sternförmiger" Struktur enthält, das erhältlich ist durch radikalische Atomtransferpolymerisation:
- eines oder mehrerer radikalisch polymerisierbarer Monomere, die ausgewählt sind unter;
(i) den Acrylestern oder Methacrylestern, die ausgehend von aliphatischen, geradkettigen, verzweigten, cyclischen und/oder aromatischen Alkoholen mit 1 bis 20 Kohlenstoffatomen hergestellt werden, wie beispielsweise Methyl(meth)acrylat, Ethyl(meth)acrylat, Propyl-(meth)acrylat, Butyl(meth)acrylat, Isobutyl(meth)acrylat und *t*-Butyl(meth)acrylat;
(ü) C₁₋₄-Hydroxyalkyl(meth)acrylaten, wie 2-Hydroxyethyl-(meth)acrylat oder 2-Hydroxypropyl(meth)acrylat;
(iii) Ethylenglykol(meth)acrylaten, Diethylenglykol(meth)-acrylaten und Polyethylenglykol(meth)acrylaten mit endständigen Hydroxy- oder Ethergruppen;
(iv) Vinylestem, Allylestern oder Methallylestern, die ausgehend von aliphatischen, geradkettigen oder verzweigten C₁₋₁₀-Alkoholen oder cyclischen C₁₋₆-Alkohlen und/oder aromatischen Alkoholen mit 1 bis 6 Kohlenstoffatomen hergestellt werden, beispielsweise Vinylacetat, Vinylpropionat, Vinylbenzoat und Vinyl-*t*-butylbenzoat;
(v) N-Vinylpyrrolidon; Vinylcaprolactam; Vinyl-N-alkylpyrrolen mit 1 bis 6 Kohlenstoffatomen; Vinyloxazolen, Vinylthiazolen; Vinylpyrimidinen; Vinylimidazolen; und Vinylketonen;
(vi) (Meth)acrylamiden, die ausgehend von aliphatischen, geradkettigen, verzweigten, cyclischen und/oder aromatischen Aminen mit 1 bis 20 Kohlenstoffatomen hergestellt werden, wie *t*-Butylacrylamid; (Meth)acrylamiden, wie Acrylamid, Methacrylamid und Di-C₁₋₄-alkyl(meth)-acrylamiden;
(vii) Olefinen, wie Ethylen, Propylen, Styrol oder substituiertem Styrol;
(viii) fluorierten oder perfluorierten Acrylmonomeren oder Vinylmonomeren, insbesondere (Meth)acrylestern mit Perfluoralkylgruppen;
(ix) Monomeren, die eine Aminogruppe in freier Form oder ganz oder teilweise neutralisiert oder ganz oder teilweise quaternisiert enthalten, wie Dimethylaminoethyl(meth)-acrylat, Dimethylaminoefhylmethacrylamid, Vinylamin, Vinylpyridin und Diallyldimethylammoniumchlorid;
(x) Carboxybetainen oder Sulfobetainen, die durch partielle oder vollständige Quatemisierung von Monomeren mit ethylenischer Doppelbindung, die eine Aminogruppe enthalten, durch Natriumsalze von Carbonsäuren mit mobilem Halogenid (beispielsweise Natriumchloracetat) oder durch cyclische Sulfone (Propansulfon) hergestellt werden;
(xi) siliconhaltigen (Meth)acrylaten oder (Meth)acrylamiden, insbesondere den (Meth)acrylestern mit Siloxangruppen; und
(xii) deren Gemischen.
in Gegenwart
- eines Initiators, der mindestens zwei durch Polymerisation radikalisch transferierbare Atome und/oder Gruppen aufweist,
- einer Verbindung, die ein Übergangsmetall enthält, das an einem Reduktionsschritt mit dem Initiator und einer "schlafenden" Polymerkette teilnehmen kann, und die unter den Verbindungen der Formel Mⁿ⁺X'ₙ ausgewählt ist, worin:
- M unter Cu, Au, Ag, Hg, Ni, Pd, Pt, Rh, Co, Ir, Fe, Ru, Os, Re, Mn, Cr, Mo, W, V, Nb, Ta und Zn ausgewählt ist,
- X' ein Halogen (insbesondere Brom oder Chlor), OH, (O)_{1/2}, eine Alkoxygruppe mit 1 bis 6 Kohlenstoffatomen, (SO₄)_{1/2}, (PO₄)_{1/3}, (HPO₄)_{1/2}, (H₂PO₄), eine Triflatgruppe, Hexafluorphosphat, Methansulfonat, Arylsulfonat, SeR, CN, NC, SCN, CNS, OCN, CNO, N₃ et R'CO₂ bedeuten kann,
wobei R eine geradkettige oder verzweigte Aryl- oder Alkylgruppe mit 1 bis 20 und vorzugsweise 1 bis 10 Kohlenstoffatomen ist und R' H oder eine geradkettige oder verzweigte Alkylgruppe mit 1 bis 6 Kohlenstoffatomen oder eine Arylgruppe bedeutet, die ggf. mit einem oder mehreren Halogenatomen und insbesondere Fluor und/oder Chlor substituiert ist; und
- n die Ladung des Metalls bedeutet; und
- eines Liganden, der ausgewählt ist unter:
(i) den Verbindungen, die mindestens ein Stickstoffatom (N), Sauerstoffatom (O), Phosphoratom (P) und/oder Schwefelatom (S) enthalten und die über eine σ-Bindung mit der Verbindung, die ein Übergangsmetall enthält, eine Koordinationsverbindung bilden können;
(ii) Verbindungen, die mindestens zwei Kohlenstoffatome aufweisen und die über eine π-Bindung mit der Verbindung, die ein Übergangsmetall enthält, eine Koordinationsverbindung bilden können;
(iii) Verbindungen, die mindestens ein Kohlenstoffatom enthalten und die über eine σ-Bindung mit der Verbindung, die ein Übergangsmetall enthält, eine Koordinationsverbindung bilden können, die jedoch mit dem Monomer bei der Polymerisation keine Kohlenstoff-Kohlenstoff-Bindung ausbilden; und
(iv) Verbindungen, die über eine µ- oder η-Bindung mit der Verbindung, die ein Übergangsmetall enthält, eine Koordinationsverbindung bilden können;
wobei das Polymer ein oder mehrere Monomere Mi enthält, deren korrespondierendes Homopolymer eine Tg von 0°C oder darüber, vorzugsweise mindestens 5°C und noch besser mindestens 20°C aufweist; wobei das Monomer Mi oder die Monomere Mi in dem fertigen Polymer in einer Menge von 55 bis 98 Gew. -%, vorzugsweise 75-95 Gew.-% und noch besser 80-90 Gew.-%, bezogen auf das Gesamtgewicht der Monomere, vorliegen; und
wobei das Polymer im Übrigen ein oder mehrere Monomere Mj enthält, deren korrespondierendes Homopolymer eine Tg von 0°C oder darunter, vorzugsweise höchstens -5°C und noch besser höchstens -10°C aufweist; wobei das Monomer Mj oder die Monornere Mj in dem fertigen Polymer in einer Menge von 2 bis 45 Gew.-%, vorzugsweise in einer Menge von 5-25 Gew.-% und noch besser in einer Menge von 10-20 Gew.-%, bezogen auf das Gesamtgewicht der Monomere, vorliegt.

2. Zusammensetzung nach Anspruch 1, wobei die Monomere ausgewählt sind unter:
- (Meth)acrylestern, die ausgehend von aliphatischen, geradkettigen oder verzweigten Alkoholen mit vorzugsweise mit 1 bis 20 Kohlenstoffatomen hergestellt werden;
- (Meth)acrylestern mit 1 bis 20 Kohlenstoffatomen, die Perfluoralkylgruppen enthalten;
- (Meth)acrylestern mit 1 bis 20 Kohlenstoffatomen, die Siloxaneinheiten enthalten;
- (Meth)acrylamiden, die ausgehend von aliphatischen, geradkettigen, verzweigten, cyclischen und/oder aromatischen Aminen, vorzugsweise mit 1 bis 20 Kohlenstoffatomen, hergestellt werden, wie *t*-Butylacrylamid; (Meth)acrylamiden, wie Acrylamid, Methacrylamid, Di-C₁₋₄-alkyl(meth)acrylamiden;
- Vinylestern, Allylestern oder Methallylestern, die ausgehend von aliphatischen, geradkettigen oder verzweigten C₁₋₁₀-Alkoholen oder cyclischen C₁₋₆-Alkohlen hergestellt werden;
- Vinylcaprolactam;
- ggf. substituiertem Styrol; und
- deren Gemischen.

3. Zusammensetzung nach einem der vorhergehenden Ansprüche, **dadurch gekennzeichnet, dass** der Initiator unter den Verbindungen der folgenden Formeln ausgewählt ist:
- R¹¹ₓ R¹²_{y} R¹³_{z} C-(RX)ₜ, in der x, y und z 0 oder eine ganze Zahl von 1 bis 4 bedeuten, t eine ganze Zahl von 1 bis 4 ist und x+y+z=4-t;
- R¹³ₓ C₆-(RX)_{y} (gesättigter Ring mit 6 Kohlenstoffatomen), wobei x eine ganze Zahl von 7 bis 11 bedeutet, y eine ganze Zahl von 1 bis 5 ist und x+y=12;
- R¹³ₓ C₆-(RX)_{y} (ungesättigter Ring mit 6 Kohlenstoffatomen),
wobei x 0 oder eine ganze Zahl von 1 bis 5 bedeutet, y eine ganze Zahl von 1 bis 6 ist und x+y=6;
- [-R¹¹)(R¹²)(R¹³)C-(RX)-]ₙ, wobei n 1 bedeutet oder darüber liegt und die Verbindung cyclisch oder geradkettig vorliegt;
- [-(R¹²)ₓC₆(RX)_{y}-R¹¹-]ₙ, wobei x 0 oder eine ganze Zahl von 1 bis 6 bedeutet, y eine ganze Zahl von 1 bis 6 ist, und n 1 bedeutet oder darüber liegt, mit x+y=4 oder 6; wobei die Verbindung cyclisch oder geradkettig ist;
- [-(R¹²)ₓC₆(RX)_{y}-R¹¹-]ₙ, wobei x 0 oder eine ganze Zahl von 1 bis 12 bedeutet, y eine ganze Zahl von 1 bis 12 ist, und n 1 bedeutet oder darüber liegt, mit x+y=10 oder 12; wobei die Verbindung cyclisch oder geradkettig ist;
- -[OSi(R¹¹)ₓ(RX)_{y}]ₙ, wobei die Verbindung cyclisch oder geradkettig vorliegt und wobei x und y 0 oder eine ganze Zahl von 1 bis 2 bedeuten und n 1 ist oder darüber liegt, mit x+y=2;
- R¹¹N-X₂
- (R¹¹)ₓP(O)_{y}-X₃₋ₓ, wobei x und y 0 oder ganze Zahlen von 1 bis 2 bedeuten und x+y=5;
- (R¹¹O)ₓP(O)_{y}-X₃₋ₓ, wobei x und y 0 oder ganze Zahlen von 1 bis 2 bedeuten, und x+y=5;
- -[(R¹¹)ₜN_{z}P(O)ₓ(O-RX)_{y}-]ₙ, wobei die Verbindung cyclisch oder geradkettig vorliegt und wobei x 0 oder eine ganze Zahl von 1 bis 4 bedeutet, y eine ganze Zahl von 1 bis 5 ist, z 0 oder eine ganze Zahl von 1 bis 2 bedeutet, t 0 oder eine ganze Zahl von 1 bis 3 ist und n 1 bedeutet oder darüber liegt;
worin bedeuten:
- die Gruppen R, R¹¹, R¹² und R¹³ unabhängig voneinander ein Wasserstoffatom; ein Halogenatom; eine geradkettige oder verzweigte Alkylgruppe mit 1 bis 20 Kohlenstoffatomen, vorzugsweise 1 bis 10 Kohlenstoffatomen und besonders bevorzugt 1 bis 6 Kohlenstoffatomen; eine Cycloalkylgruppe mit 3 bis 8 Kohlenstoffatomen; eine Gruppe -C(=Y)R⁵, -C(=Y)NR⁶R⁷ oder -R⁸₃Si; -COCl, -OH, -CN, eine Alkenyl- oder Alkinylgruppe mit 2 bis 20 Kohlenstoffatomen und vorzugsweise 2 bis 6 Kohlenstoffatomen; eine Oxiranylgruppe, eine Glycidylgruppe, eine Alkylen- oder Alkenylengruppe, die mit einer Oxiranylgruppe oder einer Glycidylgruppe substituiert ist; Aryl, Heterocyclyl, Aralkyl, Aralkenyl; eine Alkylgruppe mit 1 bis 6 Kohlenstoffatomen, in der die Wasserstoffatome ganz oder teilweise entweder durch Halogenatome, wie Fluor, Chlor oder Brom, oder durch eine Alkoxygruppe mit 1 bis 4 Kohlenstoffatomen oder durch eine Arylgruppe, eine Heterocyclylgruppe, -C(=Y)R⁵, -C(=Y)NR⁶R⁷, Oxiranyl oder Glycidyl ersetzt sind;
- X ein Halogenatom, wie Cl, Br oder I, oder eine Gruppe -OR', -SR, -SeR, -OC(=O)R', OP(=O)R', -OP(=O)(OR')₂, -OP(=O)OR', -O-NR'₂, -S-C(=S)NR'₂, -CN, -NC, -SCN, -CNS, -OCN, -CNO und -N₃, wobei R' eine Alkylgruppe mit 1 bis 20 Kohlenstoffatomen bedeutet, die ggf. mit einem oder mehreren Halogenen, insbesondere Fluor und/oder Chlor substituiert ist; und R eine geradkettige oder verzweigte Aryl- oder Alkylgruppe mit 1 bis 20 Kohlenstoffatomen und vorzugsweise 1 bis 10 Kohlenstoffatomen bedeutet; wobei die Gruppe -NR'₂ ferner eine cyclische Gruppe bedeuten kann, in der die beiden Gruppen R' so verbunden sind, dass sie einen 5-, 6- oder 7-gliedrigen Heterocyclus bilden können;
- Y O, S oder NR⁸ (vorzugsweise O),
- R⁵ eine geradkettige oder verzweigte Alkyl-, Alkylthio- oder Alkoxygruppe mit 1 bis 20 Kohlenstoffatomen; OH; OM' mit M' = Alkalimetall; eine Aryloxy- oder eine Heterocyclyloxygruppe;
- R⁶ und R⁷ unabhängig voneinander H oder eine geradkettige oder verzweigte Alkylgruppe mit 1 bis 20 Kohlenstoffatomen,
wobei R⁶ und R⁷ gemeinsam eine Alkylengruppe mit 2 bis 7 und vorzugsweise 2 bis 5 Kohlenstoffatomen bilden können;
- R⁸ H; eine geradkettige oder verzweigte Alkylgruppe mit 1 bis 20 Kohlenstoffatomen oder eine Arylgruppe.

4. Zusammensetzung nach einem der vorhergehenden Ansprüche, wobei der Initiator ausgewählt ist unter:
- Octa-2-isobutyrylbromid-octa-*t*-butyl-calix(8)aren,
- Octa-2-propionylbromid-octa-*t*-butyl-calix(8)aren, und
- Hexakis-α-brommethylbenzol.

5. Zusammensetzung nach einem der vorhergehenden Ansprüche, wobei der Ligand ausgewählt ist unter:
(i) Verbindungen der Formel: R⁹-Z-(R¹⁴-Z)ₘ-R¹⁰ oder R²⁰R²¹C[C(=Y)R⁵], worin bedeuten:
- R⁹ und R¹⁰ unabhängig voneinander Wasserstoff; eine geradkettige oder verzweigte Alkylgruppe mit 1 bis 20 und vorzugsweise 1 bis 10 Kohlenstoffatomen; eine Arylgruppe; eine Heterocyclylgruppe; eine Alkylgruppe mit 1 bis 6 Kohlenstoffatomen, die mit einer Alkoxygruppe mit 1 bis 6 Kohlenstoffatomen oder einer Dialkylaminogruppe mit 1 bis 4 Kohlenstoffatomen oder einer Gruppe -C(=Y)R⁵ oder -C(=Y)NR⁶R⁷ und/oder YC(=Y)R⁸ (wobei die Definitionen für R⁵ bis R⁸ und Y in Anspruch 3 angegeben sind) substituiert ist; mit der Maßgabe, dass R⁹ und R¹⁰ gemeinsam einen gesättigten oder ungesättigten Ring bilden können;
- die Gruppen R¹⁴ unabhängig voneinander eine zweiwertige Gruppe, die unter den Alkandiylgruppen mit 2 bis 4 Kohlenstoffatomen; den Alkenylengruppen mit 2 bis 4 Kohlenstoffatomen, den Cycloalkandiylgruppen mit 3 bis 8 Kohlenstoffatomen; den Cycloalkendiylgruppen mit 3 bis 8 Kohlenstoffatomen, den Arendiylgruppen und den Heterocyclylgruppen ausgewählt sind;
- Z O, S, NR¹⁵ oder PR¹⁵ mit R¹⁵=H, eine geradkettige oder verzweigte Alkylgruppe mit 1 bis 20 Kohlenstoffatomen, eine Arylgruppe, eine Heterocyclylgruppe; eine Alkylgruppe mit 1 bis 6 Kohlenstoffatomen, die mit einer Alkoxygruppe mit 1 bis 6 Kohlenstoffatomen oder einer Dialkylaminogruppe mit 1 bis 4 Kohlenstoffatomen oder einer Gruppe -C(=Y)R⁵ oder -C(=Y)NR⁶R⁷ und/oder YC(=Y)R⁸ (wobei die Definitionen für die Gruppen R⁵ bis R⁸ und Y in Anspruch3 angegeben sind) substituiert ist;
- wobei m im Bereich von 0 bis 6 liegt;
- R²⁰ und R²¹ unabhängig voneinander Wasserstoff; ein Halogenatom; eine geradkettige oder verzweigte Alkylgruppe mit 1 bis 20 und vorzugsweise 1 bis 10 kohlenstoffatomen; eine Arylgruppe; eine Heterocyclylgruppe; mit der Maßnahme, dass R²⁰ und R²¹ auch gemeinsam einen gesättigten oder ungesättigten Ring bilden können und mit der Maßgabe, dass jede Gruppe ferner mit einer C₁₋₆-Alkylgruppe, einer C₁₋₆-Alkoxygruppe oder einer Arylgruppe substituiert sein kann; und
(ii) Kohlenmonoxid; Porphyrinen und Porphycenen, die ggf. substituiert sind; Ethylendiamin und Propylendiamin, die ggf. substituiert sind; Multiaminen mit tertiären Aminen, wie Pentamethyldiethylentriamin; Aminoalkoholen wie Aminoethanol und Aminopropanol, die ggf. substituiert sind; Glykolen, wie Ethylenglykol oder Propylenglykol, die ggf. substituiert sind; Arenen wie Benzol, die ggf. substituiert sind; Cyclopentadien, das ggf. substituiert ist; Pyridinen und Bipyridinen, die ggf. substituiert sind; Acetonitril; 1,10-Phenanthrolin; Kryptanden und Kronenether; und Spartein.

6. Zusammensetzung nach einem der vorhergehenden Ansprüche, wobei das Polymer mit "sternförmiger" Struktur in einer Menge von 1 bis 60 Gew.-% Trockensubstanz, bezogen auf das Gesamtgewicht der Zusammensetzung, vorzugsweise 1 bis 50 Gew.-% und noch bevorzugter 5 bis 40 Gew.-% vorliegt.

7. Zusammensetzung nach einem der vorhergehenden Ansprüche, wobei mit dem Polymer mit "sternförmiger" Struktur ein Film erzeugt werden kann, der an der Oberfläche eine Härte unter 110, vorzugsweise von 1 bis 70 und noch besser 5 bis 55 aufweist.

8. Zusammensetzung nach einem der vorhergehenden Ansprüche, wobei das Polymer mit "sternförmiger" Struktur in dem Medium in gelöster Form oder in Form einer Dispersion in einer wässrigen Phase, organischen Phase, wässrig-organischen Phase, insbesondere alkoholischen oder wässrig-alkoholischen Phase vorliegt.

9. Zusammensetzung nach einem der vorhergehenden Ansprüche, die als wässrige Lösung oder Dispersion oder Lösung oder Dispersion in einem Lösungsmedium vorliegt.

10. Zusammensetzung nach einem der vorhergehenden Ansprüche, die als pflegende Grundierung für die Nägel oder Nagellack vorliegt.

11. Verfahren zur kosmetischen Behandlung der Nägel, das **dadurch gekennzeichnet ist, dass** es darin besteht, auf diese eine kosmetische Zusammensetzung nach einem der Ansprüche 1 bis 10 aufzubringen.

12. Verwendung mindestens eines in Anspruch 1 definierten Polymers in einer kosmetischen Zusammensetzung, mit der ein Film erzeugt werden kann, der gegenüber Abblättern, Stößen, Reiben, Kratzen und/oder Druck sehr beständig ist.
